# EUROPÄISCHE PATENTSCHRIFT

(11) **EP 2 326 276 B1**
(45) Veröffentlichungstag und Bekanntmachung des Hinweises auf die Patenterteilung: **30.04.2014**
(21) Anmeldenummer: 09777945.8
(22) Anmeldetag: 18.08.2009
(51) Int. Cl.: A61B 19/00

(54) **MEDIZINISCHES MESSSYSTEM UND DIE VERWENDUNG DIESES MEDIZINISCHEN MESSSYSTEMS**
MEDICAL MEASURING SYSTEM AND USE OF THIS MEDICAL MEASURING SYSTEM
SYSTÈME DE MESURE MÉDICAL ET UTILISATION DU SYSTÈME DE MESURE MÉDICAL

(30) Priorität: 18.08.2008 DE 102008038126
(43) Veröffentlichungstag der Anmeldung: 01.06.2011
(73) Patentinhaber: Naviswiss AG, 4242 Laufen (CH)
(72) Erfinder: KNOBEL, Bruno, CH-4242 Laufen (CH); FINDEISEN, Charles, CH-5430 Wettingen (CH)
(74) Vertreter: Castell, Klaus
(86) Internationale Anmeldenummer: PCT/EP2009/005979
(87) Internationale Veröffentlichungsnummer: WO 2010/020397

(56) Entgegenhaltungen:
- EP-A1- 1 415 609
- EP-A2- 0 672 389
- WO-A2-2004/002352
- WO-A2-2006/131373

## Beschreibung

Für die Computer assistierte Chirurgie (engl. Computer Assisted Surgery, CAS) werden Trackingsysteme verwendet, um die räumliche Lage und Orientierung von beispielsweise Instrumenten oder Körperteilen zu bestimmen.

Die typischen zur Zeit verfügbaren, optischen Trackingsysteme basieren einerseits auf Kamerasystemen, die in einem gewissen Abstand vom Operationsfeld stationär positioniert sind, und andererseits auf Lokatoren, die an die entsprechenden Körperteile oder Instrumente angebracht sind.

Mit dem Begriff "Trackingsystem" wird, der Notation von WO 2006/131373 A2 folgend, vorliegend eine Einrichtung beschrieben, mittels welcher ein Körper optisch ermittelt wird und eine Relativbewegung zwischen diesem und einem weiterem Körper verfolgt werden kann.

Mit dem Begriff "Navigationssystem" wird vorliegend eine Einrichtung beschrieben, bei der sowohl die Ergebnisse vom Trackingsystem als auch die räumlichen Informationen des Körpers, die mit anderen bildgebenden Verfahren wie Ultraschall, MRI oder Röntgentechnik gemessen wurden, dem Chirurgen in einem gemeinsamen Bezugssystem zur Verfügung stehen.

Mit dem Begriff "Messsystem" wird vorliegend eine Einrichtung beschrieben, die aus einem Kamerasystem besteht und weitere Komponenten wie Lichtquellen für eine Auflichtbeleuchtung, oder Projektionsmittel von strukturiertem Licht auf Objektoberflächen und /oder Videokameras und Lokatoren und/oder Strukturen mit flächenhaften Mustern beinhaltet.

Die üblicherweise eingesetzten Lokatoren bestehen aus mehreren retro-reflektierenden Kugeln, die miteinander starr verbunden sind oder aus aktiven Markern, meist bestehend aus IR-Leuchtdioden. Sowohl das Trackingsystem als auch die Lokatoren müssen eine ausreichende geometrische Grösse aufweisen, um die Messgenauigkeit des Systems zu gewährleisten. Für eine ausreichende Redundanz müssen die Lokatoren entsprechend aufwändig gestaltet werden.

Die Beleuchtung für die Lokatoren ist in der Regel im Trackingsystem integriert. Beim gegenwärtigen Stand der Technik werden die mit retro-reflektierenden Kugeln ausgestatteten Lokatoren mit infrarotem Licht angestrahlt. Die Kameraoptiken sind mit entsprechenden Farbfilter ausgerüstet. Damit werden praktisch hintergrundfreie Bilder der angestrahlten retro-reflektierenden Kugeln erhalten. Nicht relevante Details des Messvolumens sind damit ausgeblendet, was die Analyse der Kamerabilder erheblich vereinfacht.

Eine ausreichende infrarote Beleuchtung der flächenhaften Marker im Messvolumen, die keine retro-reflektierende Oberfläche haben, ist mit den zur Zeit verfügbaren Leuchtdioden kostspielig. Alternativ kann das infrarote Licht beispielsweise ganz oder teilweise von der Operationsleuchte stammen. Allerdings strahlen moderne, mit Leuchtdioden ausgestatteten Operationsleuchten nicht mehr im infraroten Bereich.

Für gewisse Anwendungen beispielsweise in der Dentalchirurgie sind die typischerweise eingesetzten optischen Trackingsysteme mit den entsprechenden Lokatoren zu gross und/oder zu unhandlich. In der internationalen Anmeldung WO 2006/131373 A2, welche als nächstes Stand der Technik angesehen wird, ist ein mobiles, handliches Trackingsystem mit direkt auf einem Instrument angebrachtem Kamerasystem beschrieben. Geeignete Strukturen und Muster sind an den Objekten angebracht.

In der WO 2006/131373 A2 ist nicht beschrieben, wie dieses Kamerasystem sterilisierbar (beispielsweise in einem Autoklaven) realisiert werden kann für den Einsatz im OP.

Des weiteren ist in der WO 2006/131373 A2 nicht beschrieben wie optische, genaue und im Autoklaven sterilisierbare Trackingsysteme in Miniaturbauweise realisiert werden können.

In der Dissertation von R.U. Thoranaghatte, Uni Bern 2008, wird ein Navigationsystem beschrieben, das aus einem mit einer Kamera ausgestatteten Endoskop und planen, auf dem Objekt angebrachten Mustern besteht. Das Messsystem hat praktisch keine Redundanz. Die Messgenauigkeit ist relativ gering: die Qualität der Messergebnisse hängt stark von der Orientierung der Muster bezüglich der des Endoskops ab. Problematisch sind verschmutzte Muster.

Flächenhafte ebene oder dreidimensionale Muster für Navigationssysteme sind bekannt. Als Beispiele seien die Veröffentlichungen US 5792147, WO 2004002352A2 oder US 6978167B2 aufgeführt. All diesen Beispielen ist gemeinsam, dass prinzipiell die kontrastreichen, mit Quadraten schachbrettartig angeordneten Muster auf wenige ausgezeichnete Punkte, nämlich die Ecken von jeweils vier benachbarten Quadraten, reduziert werden. Dieser Ansatz benötigt relativ grosse Flächen um eine bestimmte Genauigkeit des Navigationssystems zu erhalten, was die Muster relativ gross macht.

In der WO 2006/131373 A2 sind Systeme und Verfahren beschrieben, die die Vermessung der Oberflächentopologie mittels dem auf das Objekt projizierten, strukturierten Licht gestattet. Diese Systeme sind Geräte, die mit dem Trackingsystem mechanisch nicht verbunden sind. Dies kann im praktischen Einsatz nachteilig sein.

Typische optische Trackingsysteme beinhalten keine Erfassung des Operationsbereichs mittels im Trackingsystem integrierten Videokameras.

In der chirurgischen Orthopädie werden bei bestimmten Eingriffen Implantate eingesetzt, um verletzte Knochen in der korrekten Lage und Orientierung zu fixieren. Zum Fixieren werden typischerweise Schrauben verwendet, die in vorgebohrte Löcher im Knochen gesetzt werden. Die Löcher dafür müssen optimal bezüglich Eintrittsstelle und Bohrwinkel in das Knochenmaterial gebohrt werden. Oft werden für diesen Arbeitsschritt Bohrhilfen in Form von Hülsen verwendet, die temporär an den Implantaten beispielsweise mittels Schraubung befestigt sind. Die optimalen Gebiete im Knochen für die Verschraubung werden vor dem Eingriff beispielsweise mittels Röntgentechnik ermittelt.

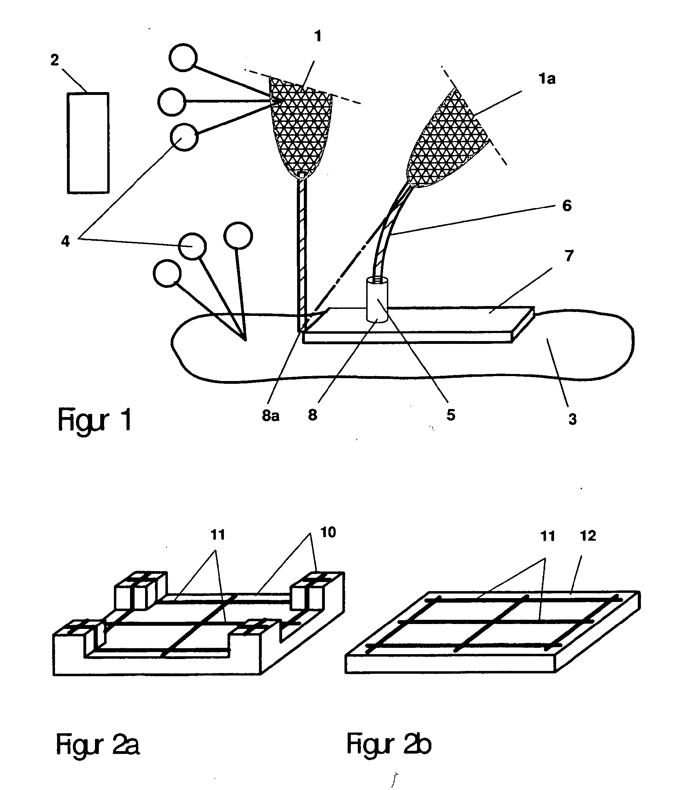

Bei CAS wird das Bohrgerät 1 mittels eines Trackingsystems 2 kontrolliert bezüglich dem Knochen 3 geführt (Figur 1). Dabei sind sowohl das Bohrgerät 1 als auch der Knochen 3 mit Lokatoren 4 ausgerüstet, so dass das Trackingsystem 2 jederzeit die relative Orientierung und Lage der Bohrgerätes 1 bezüglich dem Knochen 3 kennt. Üblicherweise werden Lage und Orientierung der am Implantat befestigten Hülsen nicht vom Trackingsystem gemessen.

Da die verwendeten Bohrer 6 relativ biegsam sind, kann die durch die am Implantat befestigte Hülse 5 definierte, tatsächliche Ansatzstelle 8 und Bohrrichtung von der geplanten Stelle 8a und Richtung abweichen, falls das Implantat 7 beispielsweise unabsichtlich verschoben wird. Die Position 1a des Bohrgeräts wird relativ zum Knochen gemessen, was aber wenig hilfreich ist, da das Ende des biegsamen Bohrers durch die vom Trackingsystem typischerweise nicht gemessene Hülse 5 geführt wird. Auch ein "allfälliges Gegendrücken" des Bohrgerätes aufgrund der visuellen Beurteilung des Chirurgen hilft nicht, die Lage und Orientierung des Schraubenlochs zu beeinflussen. So besteht die Möglichkeit, dass Schrauben und Implantate nicht optimal gesetzt werden.

Falls Bohrhülsen an Implantaten befestigt werden, liegt die Achse der Rohrführung immer normal zum Implantat und eingemittet in die Position des entsprechenden Lochs für die Bohrhülse. Für die anschliessende Bohrung ist die Bohrrichtung nicht mehr variierbar und im Falle der nicht formschlüssigen Anpassung des Implantats an die Knochenstruktur kann eine falsche Bohrrichtung resultieren.

Optische Kamerasysteme, die nur mit Kugeln ausgestattete Lokatoren erkennen, brauchen beispielsweise Taster um die Lage und Orientierung von gesetzten Implantaten zu messen. Eine weitere Methode ist die Messung bezüglich den an die Implantate temporär definiert angebrachten Lokatoren

In der chirurgischen Orthopädie werden beispielsweise künstliche Gelenkpfannen eingesetzt, deren Lage und Position nach dem Setzprozess von den typischen, optischen Trackingsystemen beispielsweise mit einem Taster erfasst werden können.

CAS mit optischen Kamerasystemen wird heutzutage aus verschiedenen Gründen nicht oder kaum für die Chirurgie von inneren Organen und Weichteilen wie Leber, Bänder oder Muskeln angewendet.

In der Patentschrift: US 6424856B1 wird eine Methode prinzipiell beschrieben, bei der CAS für Weichteile angewendet werden kann.

Die beschriebene technische Umsetzung dieser Methode basiert auf optischen Trackingsystemen, die um die Jahrtausendwende verfügbar waren. Insbesondere werden wenige diskrete Landmarken in Form von retro-reflektierenden Kugeln, die an der Oberfläche der Weichteile und an den Instrumenten befestigt sind, eingesetzt.

Da das Kamerasystem stationär in einer gewissen Entfernung positioniert ist, müssen diese Kugeln relativ gross sein. Kleine Kugeln sind vom Kamerasystem praktisch nicht messbar.

Die Genauigkeit des Systems wird durch verschmutzte Kugeloberflächen reduziert.

Zur Zeit ist nicht bekannt, dass diese Methode in der Chirurgie für Weichteile breite Anwendung hat. Offenbar sind die verfügbaren optischen Trackingsysteme mit den relativ grossen Lokatoren dafür wenig geeignet.

Im folgenden werden Vorrichtungen und Verfahren mit einem erfindungsgemäßen medizintechnischen Messsystem vorgestellt, die die oben erwähnten Nachteile nicht aufweisen oder wesentlich reduzieren.

Die Erfindung wird in den unabhängigen Ansprüchen 1 und 10 definiert. Bevorzugte Ausführungsformen werden in den abhängigen Ansprüchen beschrieben.

Das medizintechnische Messsystem weist folgende Komponenten auf: ein optisches Kamerasystem mit mindestens zwei flächenhaften, bildgebenden Sensoren, eine Lichtquelle für die Projektion von Licht auf Objekte und eine Struktur mit flächenhaftem Muster, die an einem Objekt angebracht ist.

In das medizintechnische Messsystem ist ein Kamerasystem integriert. Das Kamerasystem besteht aus mindestens zwei, bevorzugt drei Kameras mit flächenhaften Sensoren und Optik. Viele Details dazu sind in der Patentanmeldung WO 2006/131373 A2 beschrieben. Ergänzende Beschreibungen solcher Kamerasysteme sind in der vorliegenden Schrift enthalten.

Die Muster auf den Strukturen, die auf Objekten aufgebracht sind, werden vom Kamerasystem gemessen, analysiert und mittels in der Photogrammetrie bekannten Verfahren räumlich beschrieben.

Mit dem Begriff "Struktur" wird, der Notation von WO 2006/131373 A2 folgend, im Sinne der Patentanmeldung ein dreidimensionales Gebilde beschrieben, welches ein Muster aufweist. Eine Struktur ist vorliegend auch ein Körper im Sinne der Patentanmeldung. Die Struktur ist beispielsweise als ebene Platte oder als stumpfe Pyramide ausgebildet.

Ein "Muster" ist, der Notation von WO 2006/131373 A2 folgend, im Sinne der Patentanmeldung ein Bild, welches beispielsweise aus geraden oder gekrümmten Linien unterschiedlicher Breite und Länge, aus Kreisen, aus Ellipsen, aus Dreiecken und aus Rechtecken oder aus Kombinationen daraus besteht. Es ist vorzugsweise direkt auf eine Oberfläche eines Körpers, wie einer Struktur, aufgebracht. Wesentlich ist hierbei, dass sich das Muster kontrastreich von einer Oberfläche unterscheidet, auf welche es angebracht ist.

Eine besonders bevorzugte Ausführung des medizintechnischen Messsystems ist, dass die Struktur mit flächenhaftem Muster an einem Objekt angebracht ist.

Eine besonders bevorzugte Ausführung des medizintechnischen Messsystems ist, dass die Strukturen bioresorbierbar sind.

Eine besonders bevorzugte Ausführung des medizintechnischen Messsystems ist, dass die flächenhaften Muster aus verschiedenfarbigen Gebieten bestehen.

Die Oberflächenbeschaffenheit der Strukturen mit aufgebrachten Mustern ist von grosser Wichtigkeit. Im folgenden werden dazu einige wesentliche Aspekte beschrieben.

Das auf die mit kontrastreichem Muster ausgestattete Oberfläche eingestrahlte Licht wird zurückgestreut. Das rückgestreute Licht soll idealerweise Lambert - Charakteristik aufweisen.

Die das Licht absorbierenden, mit dem heutigen Stand der Technik herstellbaren Musterteile dürfen idealerweise keinen vom Kamerasystem messbaren Glanz aufweisen oder optische lokale Strukturen zeigen.

Allfällige durch das Material oder den Herstellprozess sichtbare Strukturen der Oberflächen wie lokaler Glanz dürfen das Kamerasystem nicht stören. Insbesondere eignen sich retro-reflektierende Oberflächen aufgrund ihrer mechanischen und optischen Eigenschaften nicht, falls das Kamerasystem Details der Oberfläche wie die Glaskugeln oder Reflektor - Pyramiden auflösen kann.

Sekundäre Muster und/oder Strukturen sind, der Notation von WO 2006/131373 A2 folgend, im Sinne der Patentanmeldung von weiteren nichtoptischen bildgebenden Systemen wie Ultraschall, MRI, CT oder C-Bogen erkennbar. Sekundäre Muster und/oder Strukturen sind räumlich definiert angeordnet bezüglich den vom optischen Kamerasystem messbaren Mustern. In einer bevorzugten Ausführung sind die kontrastreichen Muster auf der Oberfläche einer Struktur aufgebracht, die selber sekundäre Muster und/oder Strukturen enthält.

Eine besonders bevorzugte Ausführung des medizintechnischen Messsystems ist, dass die Struktur mit flächenhaftem Muster, geometrische und sekundäre, von nichtoptischen bildgebenden Systemen wie einem Ultraschallgeräte, MRI und/oder Röntgensysteme erkennbare, Muster aufweist.

Kamerasysteme, die fähig sind flächenhafte Strukturen im Messvolumen mit zahlreichen anderen sichtbaren Objekten aufzufinden und zu analysieren, sind mit einer Beleuchtung im sichtbaren Bereich einsetzbar. Einige wesentliche Aspekte für die Verwendung von weissem Licht werden im folgenden dargestellt.

Das weisse Licht kann einerseits von der Operationsleuchte selber stammen. Zusätzlich kann eine Aufhellbeleuchtung von einer in das Messsystem beispielsweise integrierten Matrix mit Leuchtdioden mit weissem Spektrum die Lichtintensität im Messvolumen erhöhen. Damit werden Bilder mit kontrastreich abgebildeten Mustern erzeugt. Die Aufhellbeleuchtung wird beispielsweise während einer kurzen Belichtungszeitspanne des Sensors eingesetzt.

Eine besonders bevorzugte Ausführung des medizintechnischen Messsystems ist, dass es Leuchtmittel zum gepulsten Aufhellen des Objektes beinhaltet.

Das Spektrum der Aufhellbeleuchtung kann beispielsweise das gleiche wie dasjenige der Operationsleuchte sein. Alternativ kann das Aufhellspektrum farbig sein, solange dies den Chirurgen nicht stört.

Die Intensität und/ oder Pulsdauer der Aufhellbeleuchtung wird vom Kamerasystem selber dynamisch aufgrund der Bildanalysen bestimmt und eingestellt.

Die Objektive der Kameras können mit optischen Bandpassfiltern ausgerüstet sein. Damit wird beispielsweise der spektrale Bereich bevorzugt, der einer maximalen Sensitivität der vorzugsweise monochromatischen Kamerasensoren entspricht. Ein weiterer Vorteil der optischen Bandpassfilter ist, dass die Farbkorrektur der Objektive nur im selektierten Spektrum des weissen Lichts optimal sein muss, was die Verwendung von preiswerten Objektiven gestattet.

Die Lichtintensität der Operationsleuchte kann eine Welligkeit von beispielsweise 100 Hz oder 120 Hz aufweisen, die von deren Energieversorgung verursacht wird. Deshalb kann es vorteilhaft sein, einen Lichtsensor mit Auswerteeinheit im Messsystem zu integrieren, der beispielsweise bevorzugt im Maximum der Lichtintensität die Synchronisationseinheit des Messsystems für jede neue Bildsequenz anstösst.

Optional verwendetes strukturiertes Licht, das auf Objekte im Messvolumen projiziert wird, soll von den optischen Bandpassfiltern durchgelassen werden.

Aus der Photogrammetrie ist bekannt, dass die räumliche Lage und Orientierung von Strukturen mit dreidimensional angeordnetem, flächenhaften Muster vom Kamerasystem prinzipiell mit höherer Genauigkeit ermittelt werden als von Strukturen mit planar angeordnetem Muster. In den Figuren 2a und 2b sind schematisch zwei Strukturen mit gleichem Muster 11, einerseits dreidimensional 10 auf zwei Stufen und andererseits planar 12 auf einer einzigen Ebene angeordnet, dargestellt.

Beim Design der Muster können sich kreuzende Linien mit optimalen Längen und Breiten vorteilhaft sein, da erstens der Kreuzungspunkt mit erhöhter Genauigkeit mittels geeigneter Algorithmen bestimmt werden kann (verglichen mit nur einem Kreis mit Durchmesser entsprechend den Liniendicken) und zweitens redundante Information vorhanden ist, um eine mögliche Verschmutzung der Muster erkennen und teilweise kompensieren zu können. Optional kann das Gebiet des Kreuzungspunktes im Muster weggelassen werden.

Die dreidimensional angeordneten Muster können aus stückweise ebenen Mustern 10 bestehen. Alternativ können sie vollständig auf einer beliebigen, für das Kamerasystem geeigneten Oberflächentopologie angeordnet sein.

Damit können beispielsweise Strukturen mit dreidimensional angeordnetem, geometrischem Muster mit kleinerer Fläche realisiert werden als Strukturen mit planar angeordnetem Muster bei sonst gleicher Genauigkeit bei der Bestimmung von Lage und Orientierung der Muster.

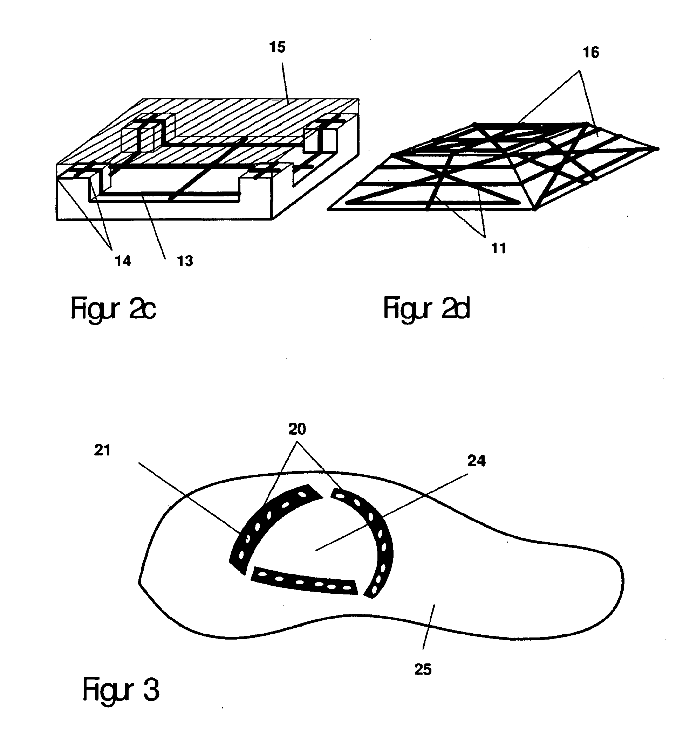

Die mit Mustern versehene dreidimensionale Struktur kann mit einer transparenten Schutzschicht 15 überzogen sein, so dass das gesamte Muster bedeckt und die Strukturoberseite planar und von ausreichender optischer Qualität ist (in Figur 2c schematisch teilweise angedeutet). Der Algorithmus des Messsystems berücksichtigt den optischen Effekt der transparenten Schutzschicht unter verschiedenen Betrachtungswinkeln.

Diese Schutzschicht schützt einerseits das dreidimensionale Muster 13 vor Kratzern und Verschmutzung, andererseits verhindert sie beispielsweise eine Beschädigungen der sterilen Handschuhe des Chirurgen durch scharfe Kanten und Ecken.

Eine weitere Ausführung einer dreidimensionalen Struktur ist beispielsweise in Form einer stumpfen Pyramide von quadratischer Grundfläche (Figur 2d). Jede der fünf Seitenflächen 16 kann ein planares oder auch ein dreidimensionales Muster enthalten. Die Pyramidengrundfläche liegt dabei auf dem Objekt auf. Optional kann eine entsprechende transparente Schutzschicht aufgebracht sein.

Eine offenbarte Ausführung des medizintechnischen Messsystems ist, dass die Struktur mit flächenhaftem Muster, geometrische Muster auf starren flachen oder starren dreidimensionalen Strukturen aufweist.

Eine weitere offenbarte Ausführung von Mustern auf Strukturen ist, dass die Prothesen oder Implantate in geeigneter Weise mit Mustern ausgestattet werden. Aufgrund der vom Kamerasystem analysierten Muster werden zusätzliche mechanische Eigenschaften der Prothesen oder Implantate erhalten. Beispielsweise wird mit einer Messung die räumliche Position des Kugelzentrums der implantierten künstlichen Hüftgelenkpfanne bezüglich einem auf dem Pelvis fixierten Muster bestimmt.

Eine offenbarte Ausführung des medizintechnischen Messsystems ist, dass die flächenhaften Muster direkt auf Implantaten, Prothesen, Fräsköpfen und/oder Raspeln angebracht sind.

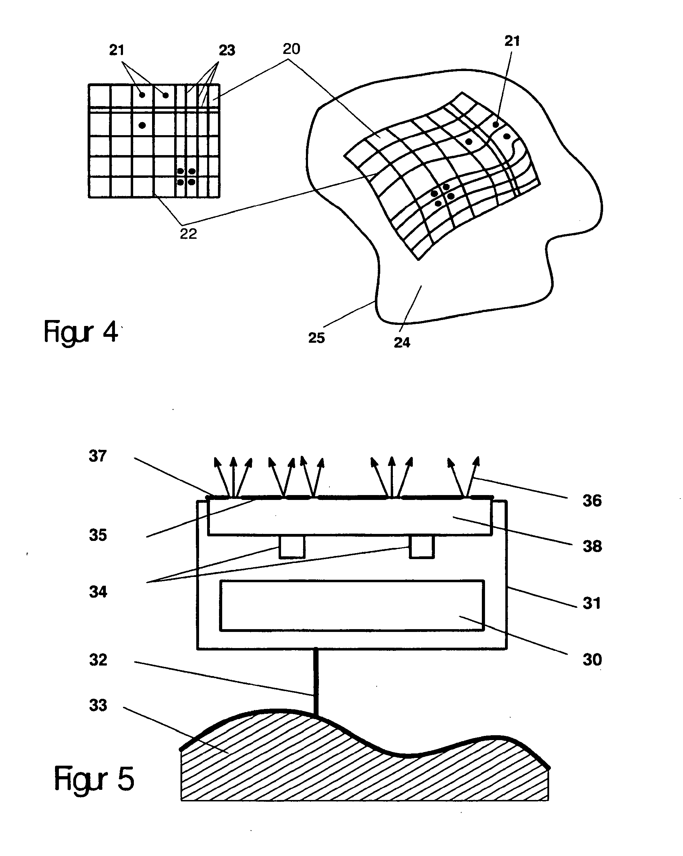

Erfindungsgemäß werden Strukturen mit optisch kontrastreichen geometrischen Mustern auf flexibler und dehnbarer Folie aufgebracht. Solche Muster sind in den Figuren 3 und 4 beispielhaft dargestellt. Diese Muster sind beispielsweise Kreisflächen 21 auf einem Streifen mit dieser flexiblen Folie 20, ein Gitter 22 von sich kreuzenden Linien 23 oder eine Punktmatrix 21. Wie in den Figuren 3 und 4 beispielhaft dargestellt, werden diese Folien auf die Oberfläche 24 der vorzugsweise deformierbaren Objekte 25 aufgebracht. Zur Orientierung und Identifikation des Musters oder Teilen davon dienen beispielsweise einige ausgezeichnete geometrische Elemente oder nicht äquidistant angeordnete geometrische Elemente.

Die flexible Folie kann in einer bevorzugten Ausführung als Schlauch ausgebildet sein, der über die entsprechenden Objekte gelegt wird.

Ein Merkmal des medizintechnischen Messsystems ist, dass die Muster auf flexiblen, verformbaren Strukturen angeordnet sind.

Auch nur teilweise vom Kamerasystem einsehbare Mustergebiete reichen aus, um die sichtbare relevante Oberflächentopologie zuverlässig zu ermitteln.

Das aufgebrachte Muster ist ausreichend redundant. So werden verschmutzte Gebiete vom Kamerasystem zuverlässig erkannt und bei der Bestimmung der Oberflächentopologie entsprechend berücksichtigt. Gegebenenfalls kann eine entsprechende Information dem Chirurgen übermittelt werden wie beispielsweise eine Aufforderung zum Reinigen der Folie.

Für einige Anwendungen ist es vorteilhaft, wenn die flexible Folie zusätzlich zu den optisch kontrastreichen, flächenhafte Formen sekundäre beispielsweise röntgen - opake Gebiete enthält, deren Lage bezüglich den geometrischen Formen bekannt ist. Somit kann beispielsweise mittels Röntgentechnik in der Leber die sekundären Gebiete in der Folie bezüglich der freigelegten Leber registriert werden. Damit sind die optisch kontrastreichen, flächenhafte Formen ebenfalls registriert.

Die Folie kann Gebiete enthalten, die mittels Ultraschalltechnik vermessen werden können. Die Lage dieser Gebiete ist bezüglich den geometrischen Mustern bekannt.

Ein besonders bevorzugtes Verfahren für einen chirurgischen Eingriff an einem Körperteil mit dem medizintechnischen Messsystem ist, dass die mittels strukturiertem Licht vermessene Körperoberfläche bezüglich den mit anderen bildgebenden Verfahren wie MRI, Ultraschall- oder Röntgentechnik erfassten Körperdaten registriert wird.

In einer weiteren Realisierung der Vorrichtung kann eine einzelne Folie mit vielen geometrischen Formen durch eine Vielzahl von separaten Folien mit einfachen geometrischen Formen wie beispielsweise Kreisen ersetzt werden. Diese separaten Folien 20 werden, wie in der Figur 3 schematisch dargestellt, einzeln auf die Oberfläche der freigelegten Leber angebracht.

Das Anbringen der flexiblen Strukturen wie beispielsweise von Folien auf das Objekt erfolgt beispielsweise mittels geeignetem biokompatiblem Klebstoff.

Das aufgebrachte Muster kann beispielsweise eine Kombination mit starren Gebieten für die Bestimmung der Lage und Orientierung sowie Identifizierung und flexiblen Gebieten mit einfachen Mustern für die Konturbestimmung des Objekts sein.

Zusätzlich kann das mobile Messsystem mit starr montierten chirurgischen Instrumenten ausgerüstet sein. Das Kamerasystem orientiert sich an den am Organ angebrachten Strukturen. Das Instrument kann beispielsweise ein laparoskopisches Instrument sein. Es kann auch ein weiteres Messsystem wie ein Ultraschallmesskopf, ein Taster, ein Kraftmesssystem sein.

Aktive, das heisst selbstleuchtende, flächenhafte Strukturen senden Lichtsignale aus, die vom Kamerasystem empfangen und analysiert werden. Die Signale liegen typischerweise im weissen oder infraroten Spektrumsbereich. Die Energieversorgung ist aus praktischen Gründen in den Strukturkörper (im folgenden auch "Marker" benannt) integriert. Wie in der Figur 5 dargestellt, sind dies beispielsweise handelsübliche Knopfzellen 30, die nach Bedarf in den Strukturkörper 31 eingesetzt werden. Der Strukturkörper wird mittels geeigneter Halterung 32 am Objekt 33 starr befestigt.

Das Licht der aktiven Struktur wird beispielsweise von in den Strukturkörper integrierten Leuchtdioden (LED) 34 oder einer Leuchtdiodenmatrix generiert. Damit wird das in den Strukturkörper integrierte geometrische Muster 35 beleuchtet. Nur an den geometrisch definierten Stellen tritt das Licht 36 hinaus.

Die vom Muster abgedeckten Bereiche 37 absorbieren ebenfalls das auf die Struktur auftreffende externe Licht, das beispielsweise von der Operationsbeleuchtung stammt. Damit bleibt der Kontrast vom abgebildeten Muster für das Kamerasystem ausreichend.

Ein Homogenisator 38 erzeugt dabei eine konstante Leuchtdichte des vom Muster durchgelassenen Lichts. Ein Homogenisator ist beispielsweise ein optisches Element mit Hologrammstruktur oder ein DOE (Diffractive Optical Element).

Anstelle der LEDs und des Homogenisators kann eine leuchtende Fläche, die beispielsweise mittels OLED (Organische Leuchtdioden) mit dem Muster realisiert ist, eingesetzt werden. Das bevorzugte Emissionsspektrum liegt dabei im weissen, im farbigen (grün, gelb oder rot) oder im nahen infraroten Bereich.

Die aktiven Strukturen sind für den praktischen Einsatz in der Chirurgie konstruiert. Insbesondere müssen sie sterilisierbar sein. In einer bevorzugten Ausführung werden Batterien und aktive elektronische Elemente wie LED und Stromversorgung mittels geeigneten Vorrichtungen kurz vor Gebrauch in das sterile Gehäuse der aktiven Struktur eingesetzt. In einer weiteren bevorzugten Ausführung werden Batterien, LED etc. für hohe Temperaturen eingesetzt. Damit ist die Sterilisierung der gesamten aktiven Struktur möglich.

Die aktiven Strukturen können für einmaligen Gebrauch vorgesehen sein, was die Logistikkette vereinfacht. Die Strukturen können auch für mehrmaligen Gebrauch konzipiert sein.

Die aktive Struktur gemäss Figur 5 erzeugt ein Lichtmuster mit konstantem Lichtstrom. Das Kamerasystem nutzt das von seiner Optik gesammelte Licht jedoch nur während der Sensorintegrationszeit. Daher wird die Batterie der aktiven Struktur unnötig belastet, was die Einsatzdauer der aktiven Struktur reduziert. Der Lichtstrom der aktiven Struktur muss im gesamten Messvolumen und für jede Orientierung ausreichend sein. Dies bedingt, dass deshalb zu viel Licht von der aktiven Struktur generiert werden muss. Auch dies belastet die Batterie.

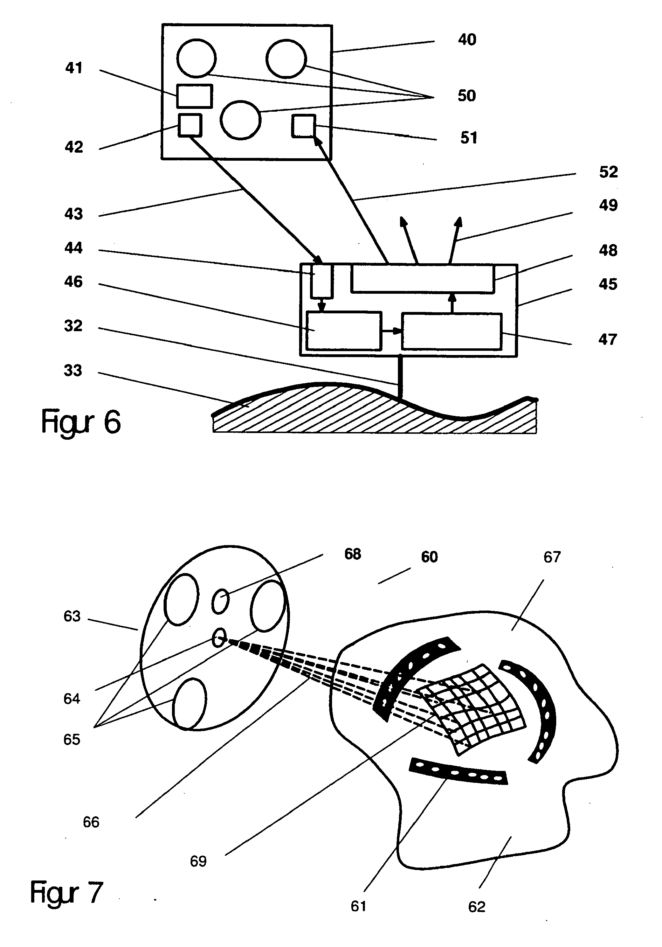

In der Figur 6 wird beispielhaft gezeigt, wie die Lichterzeugung der aktiven Struktur vom Messsystem 40 aus gesteuert und synchronisiert wird. Das Synchronisationsmodul 41 vom Messsystem generiert die Integrationszeiten der Kamerasensoren und die Pulssequenzen für die Signal-LED 42. Das Lichtsignal 43 gelangt zum Lichtempfänger 44 der aktiven Struktur 45. Ein lokales Auswertemodul 46 analysiert die Lichtsignale 43 und generiert im Leistungsmodul 47 das entsprechende Signal für die Leuchteinheit 48 mit dem integrierten Muster. Die Leuchteinheit generiert den Lichtstrom 49, der teilweise von den Optiken 50 des Kamerasystems 40 gesammelt und analysiert wird. Der optionale Lichtempfänger 51 des Messsystems unterstützt das Synchronisationsmodul 41 mit zusätzlichen Informationen vom Sichtsignal 52.

Der Lichtstrom-Puls der aktiven Struktur muss keineswegs konstant sein bezüglich Intensität und Zeitdauer. Der Lichtstrom der aktiven Struktur kann sich für optimale Kamerabilder sowohl in der Intensität als auch in der Dauer ändern. Typischerweise sind diese Grössen von der Orientierung und vom Ort der aktiven Struktur innerhalb des Messvolumens abhängig.

Die Variationen sowohl von der Intensität als auch in der Pulsdauer ändern sich für eine bestimmte Struktur während einer Serie von Messsequenzen wenig. Deshalb müssen nicht für jede Aufnahme des Kamerasystems die Bedingungen erneut an die aktive Struktur übermittelt werden. So reicht es beispielsweise aus, die Lichtbedingungen für eine aktive Struktur beispielsweise nach zehn Aufnahmen neu zu übermitteln. Mehrere aktive Strukturen können mit dynamisch einstellbarem Lichtstrom im Messvolumen gleichzeitig eingesetzt werden.

Die zeitliche Abfolge der entsprechenden Steuersignale der Signal LED eines Pulses ist beispielsweise ein eindeutiges Signalmuster für den Start einer neuen Messung, gefolgt von Pulssequenzen für die Identifikationsnummer einer aktiven Struktur und der verlangten Intensität und Dauer des von der aktiven Struktur ausgesandten Lichts.

Der Lichtempfänger mit der (nicht in der Figur 6 eingezeichneten) Auswerteeinheit bestimmt während der Messung mit dem Messsystem typische Lichtintensitäten des Messvolumens. Zusätzlich ist es möglich den Status der einzelnen aktiven Struktur sequentiell in einem Diagnose-Modus abzufragen. Dabei wird das Licht der aktiven Struktur entsprechend moduliert, vom Lichtempfänger des Messsystems empfangen und von dessen Auswerteeinheit analysiert.

Optional sind diese aktiven Strukturen mit einer Standby-Schaltung ausgerüstet, so dass sie nur nach Bedarf aktiviert werden. Damit wird die Energieversorgung entsprechend geschont. Auch wird die logistische Handhabung der aktiven Struktur wesentlich vereinfacht.

Der konstruktive Aufbau der aktiven Struktur erlaubt es bildgebenden, mit Röntgenstrahlung arbeitenden Systemen, die räumliche Position und Lage als auch die Identifikation der aktiven Struktur zu ermitteln. Dazu muss beim konstruktiven Aufbau berücksichtigt werden, dass die röntgen-opaken (Batterien, Leiterplatten, Befestigungsschrauben) und röntgen-transparenten (Kunststoffe) Materialien entsprechend räumlich angeordnet werden. Diese Anordnung ist in einem festen geometrischen Bezug zum Muster (beispielsweise das Muster in Figur 5).

Die aktiven Strukturen können sowohl mit planen Mustern als auf mit dreidimensionalen Mustern wie beispielsweise einer Pyramide ausgerüstet sein.

Die Muster der aktiven Struktur können mittels Steuersignalen bedarfsweise konfiguriert werden. Dazu sind beispielsweise aktive Strukturen mit OLED - Muster geeignet. In einer bevorzugten Ausführung sind die aktiven Strukturen ganz oder teilweise flexibel.

Eine besonders bevorzugte Ausführung des medizintechnischen Messsystems ist, dass aktiv strahlende Strukturen vorgesehen sind.

Eine besonders bevorzugte Ausführung des medizintechnischen Messsystems ist, dass die aktiven Strukturen eine autonome Energieversorgung haben.

Die Vorrichtung des Messsystems 60 besteht, wie in der Figur 7 beispielhaft aus flächenhaften Mustern auf flexiblen Folien 61 auf deformierbarem Objekt 62 dargestellt, aus einem optischen, mobilen Kamerasystem 63 mit den Optiken 65, das die räumliche Orientierung und Position von flächenhaften Mustern misst, und einer in das Messsystems integrierten Lichtquelle 64, die strukturiertes Licht 66 auf die Oberfläche 62 des mit flächenhaften Strukturen 61 ausgerüsteten Objekts 67 projiziert. Die Richtung des emittierten strukturierten Lichts 66 ist bezüglich dem Kamerasystem 63 definiert. In einer weiteren bevorzugten Ausführung ist diese Lichtquelle ein Laser, dessen Lichtstrahl beispielsweise mittels einer Matrix aus Mikrolinsen strukturiert wird. In einer weiteren bevorzugten Ausführung ist diese Lichtquelle ein Laser, dessen Lichtstrahl beispielsweise mittels einem DOE (diffractive optical element) strukturiert wird. In einer weiteren bevorzugten Ausführung wird das DOE durch einen Scanner mit mindestens einem definiert kippbaren Spiegeln ersetzt. Damit können zeitlich variierende Lichtstrukturen auf das Objekt 67 projiziert werden. In einer weiteren bevorzugten Ausführung wird das DOE durch eine dynamisch konfigurierbare Spiegelmatrix ersetzt. Damit können variierende Lichtstrukturen auf das Objekt 67 projiziert werden. In einer weiteren bevorzugten Ausführung werden mehrere verschiedenfarbige Lichtquellen mit strukturiertem Licht in das Kamerasystem, das mit Farbsensoren ausgerüstet ist, eingebaut. Damit besteht die Möglichkeit, dass die jeweils optimale Lichtquelle für die Oberflächentopologiemessung eines Objekte verwendet wird.

Eine besonders bevorzugte Ausführung des medizintechnischen Messsystems ist, dass es mindestens eine weitere Lichtquelle für die Projektion von strukturiertem Licht auf Objekte aufweist.

Eine besonders bevorzugte Ausführung des medizintechnischen Messsystems ist, dass die weitere Lichtquelle eine zu der ersten unterschiedliche Farbe aufweist.

Eine besonders bevorzugte Ausführung des medizintechnischen Messsystems ist, dass die weitere Lichtquelle eine zu der ersten unterschiedliche Struktur projiziert.

Eine besonders bevorzugte Ausführung des medizintechnischen Messsystems ist, dass für die Projektion von strukturiertem Licht auf Objekte ein Lichtstrahl auf mit dem Kamerasystem starr verbundene kontrolliert bewegliche Spiegel projiziert wird.

Eine besonders bevorzugte Ausführung des medizintechnischen Messsystems ist, dass für die Projektion von strukturiertem Licht auf Objekte Laserlicht auf diffraktive optische Elemente (DOE) projiziert wird.

Der Laserstrahl wird auf die mit Strukturen ausgestattete Objektoberfläche innerhalb des Messbereichs vom Messsystem gerichtet. In der Figur 7 sind beispielhaft einige Lichtstrahlen 66 des strukturierten Lichts eingezeichnet. Damit wird auf der Oberfläche des Objekts ein im allgemeinen dreidimensionales Lichtmuster 69 erzeugt.

Das an der Oberfläche gestreute strukturierte Licht wird vom Kamerasystem gemessen, analysiert und in räumlichen Bezug zu den Mustern auf den benachbarten Strukturen auf dem Objekt gesetzt.

Durch Änderung der räumlichen Lage und Orientierung des Messsystems wird das Muster mit dem strukturierten Licht entlang der Oberfläche bezüglich der Strukturen verschoben. Damit wird die Oberflächentopologie innerhalb des gewünschten Bereichs bezüglich den benachbarten Strukturen ermittelt. Zeitliche Veränderungen der Oberflächentopologie sind mit dieser Methode messbar.

Ein besonders bevorzugtes Verfahren für einen chirurgischen Eingriff an einem Körperteil mit dem medizintechnischen Messsystem ist, dass die Oberfläche eines zu behandelnden Körperteils lokal mittels strukturiertem Licht vermessen wird und in räumlichen Bezug zu einer angebrachten Struktur mit Muster gesetzt wird.

Ein besonders bevorzugtes Verfahren für einen chirurgischen Eingriff an einem Körperteil mit dem medizintechnischen Messsystem ist, dass ein Teil oder die ganze Oberfläche von Gelenkknorpel lokal mittels strukturiertem Licht dreidimensional vermessen wird und damit Ort und Grösse von abgenützten Gelenkteilen lokalisiert werden.

Ein besonders bevorzugtes Verfahren für einen chirurgischen Eingriff an einem Körperteil mit dem medizintechnischen Messsystem ist, dass die starren Strukturen oder flexiblen Folien mit geometrischen Mustern anatomische Strukturen und anatomische gegebene Muster sind.

Optional sind wie schematisch in der Figur 7 dargestellt eine oder mehrere Videokameras 68 in das Messsystem integriert. Die Aufgaben der Videokamera sind Bilderfassung des relevanten Operationsbereichs beispielsweise für die übersichtliche Darstellung zu Navigationszwecken auf einem im Messsystem integrierten Bildschirm zur Erkennung von Objekttextur der Oberflächen, Objektstrukturen wie Blutgefässen und/oder von Form und Lage von Implantaten als Warnvorrichtung für Korrelationsanalysen von aufeinanderfolgenden Bildern und/oder für Dokumentationszwecke.

In einer weiteren offenbarten Ausführung der Vorrichtung befinden sich auf starren Objekten starre Muster auf starren Strukturen.

In einer weiteren bevorzugten Ausführung der Vorrichtung im medizintechnischen Messsystem eine Anzeigevorrichtung wie ein Monitor mit Bedienelementen integriert.

Eine besonders bevorzugte Ausführung des medizintechnischen Messsystems ist, dass es mindestens eine mit dem Kamerasystem starr verbundene Anzeigeeinheit mit Bedienelementen aufweist.

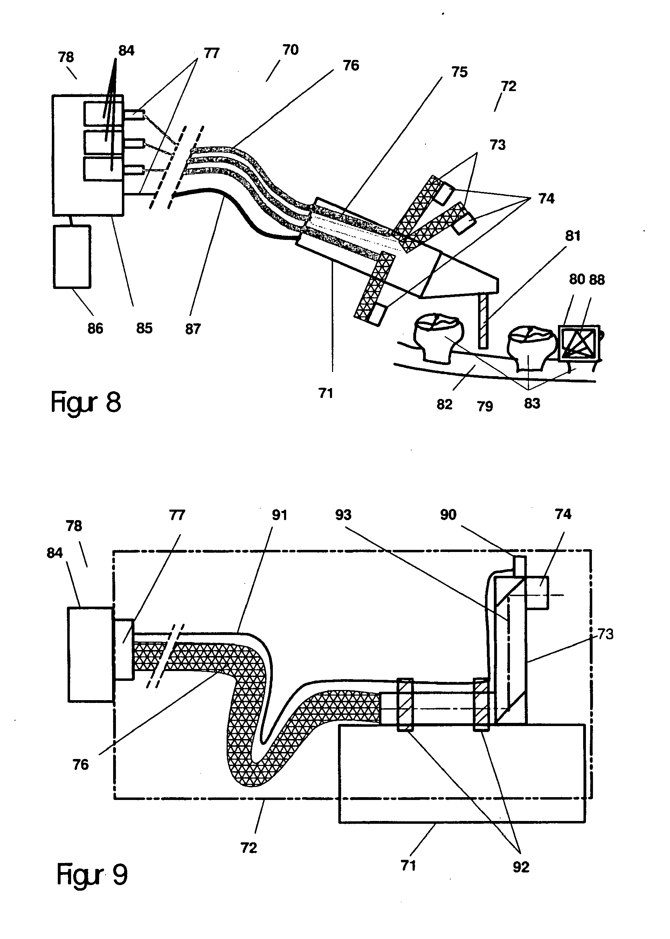

Die in der Figur 8 schematisch dargestellte Vorrichtung zeigt ein Kamerasystem 70, das aus zwei Teilen 72 und 78 besteht. Das vorgestellte Beispiel betrifft das Gebiet der dentalen Chirurgie mit einem Bohrgerät 71 als Werkzeug; es ist im Prinzip auch beispielsweise in der chirurgischen Orthopädie oder in anderen chirurgischen Gebieten einsetzbar.

Der erste, sterile Teil 72 umfasst mehrere Periskope 73 mit Optik und Beleuchtungsvorrichtung 74, Bildleiter 75 entlang dem Werkzeug 71, einen flexiblen und ausreichend langen Bildleiter 76 und Bildleiteradapter 77 zum zweiten nichtsterilen Teil 78 mit den Sensoren 84, Auswerteeinheit 85 und Host 86. Die Anzahl der Periskope ist mindestens zwei. Im folgenden wird die Variante mit drei Periskopen beschrieben, da dies markante Vorteile bezüglich Genauigkeit und Redundanz und damit Sicherheit gegenüber herkömmlichen Trackingsystemen bedeutet. Weitere Verbindungskabel 87 versorgen den ersten Teil 72 mit Energie, Wasser oder Pressluft.

Die Optiken der Periskope sehen das Operationsfeld 79 ein. Insbesondere müssen sie die Strukturen 80 mit dem geometrischen Muster 88 während dem Eingriff sehen, damit die räumliche Orientierung und Position des Werkzeugs 71 mit dem Bohrer 81, der Bohrstelle 82 und der Zähne 83 bestimmt werden können.

Der Bildleiter 75 ist vorzugsweise in endoskopischer Bauweise ausgeführt. Der Bildleiter hat die Aufgabe, das von der Optik auf dem Periskop eingesehene Operationsfeld 79 zum entsprechenden Sensor 84 zu übertragen.

Die Beleuchtungsvorrichtung kann, wie beispielsweise in der Figur 9 schematisch dargestellt, aus LEDs 90 mit weissem Licht bestehen, die in geeigneter Weise an den Periskopen 73 mit den optischen Achsen 93 angebracht sind und mittels Kabeln 91 mit Energie versorgt werden. Die Beleuchtung kann auch direkt über Lichtleiter und Optik erfolgen, wobei sich die LEDs im zweiten Teil des Messsystem befinden.

Die Periskope sind mechanisch in definierter Weise mit dem Werkzeug, beispielsweise mit einem Aufsteckmechanismus 92, verbunden. Mehrere definierte Befestigungspositionen können dafür vorgesehen sein, damit die Optiken der Periskope optimal auf das Arbeitsgebiet ausgerichtet sind.

Dieser erste Teil des Kamerasystems kann modular aufgebaut sein, was mehrere Optionen ermöglicht.

In einer bevorzugten Ausführung kann die Optik von jedem Periskop auswechselbar sein, so dass das Karnerasystem optimal auf das Arbeitsfeld bezüglich Abstand und Sehfeld ausgerichtet ist. Eine weitere Option ist, dass die Periskope samt Optiken auswechselbar sind. Eine weitere Option ist die auswechselbare Basis der Periskope, so dass die Optiken einen ausreichenden geometrischen Abstand zueinander haben. Denn bei grösserem Arbeitsvolumen sind in der Regel die Optiken entsprechend weiter auseinander angeordnet. Damit ist die spezifizierte Genauigkeit des Kamerasystems immer gewährleistet.

Dieser erste Teil 72 ist in sterilisierbarer Bauweise ausgeführt. Die übliche durchgeführte Sterilisationsart ist mittels Autoklaven oder auch mittels Gassterilisation. Wichtig dabei ist, dass der erste Teil des Kamerasystems ausreichend steril für den Einsatz im Operationsfeld ist.

Eine besonders bevorzugte Ausführung des medizintechnischen Messsystems ist, dass beim Kamerasystem zwischen Sensor und Optik jeweils ein Periskop angeordnet ist.

Der zweite Teil 78 umfasst einen Sensor 84 für jede Periskopeinheit, eine in der Figur 9 nicht dargestellte Auswerteeinheit und Visualisierungseinrichtung. Dieser zweite Teil braucht nicht autoklavierbar zu sein, da er ausreichend weit vom Operationsfeld positioniert werden kann. Jedoch kann das Gehäuse dieses Teils beispielsweise mit entsprechenden Chemikalien, beispielsweise mit Ethylenoxid, äusserlich sterilisiert werden.

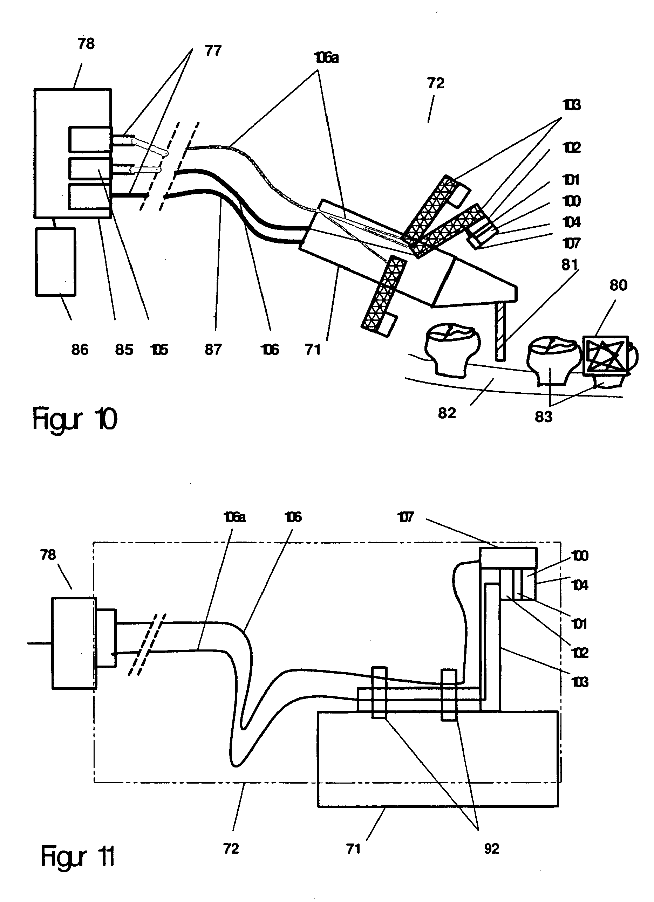

Alternativ können, wie schematisch in den Figuren 10 und 11 beispielhaft dargestellt, im sterilen Teil 72 Optik 100, optische Hochpassfilter 104, Sensoren 101 und ein Teil der Elektronik 102 ganz vorne an einer Halterung 103 angeordnet sein. Der Bildleiter entfällt. Das Licht für die Beleuchtung wird in dieser Vorrichtung im nichtsterilen Teil 78 mit dem Beleuchtungsmodul 105 erzeugt und über Lichtleiter 106 zur Beleuchtungsoptik 107 geführt. Die lokal aufbereiteten Sensorinformationen werden in diesem Beispiel an die Elektronik im nichtsterilen Teil 78 über die Kabel 106a gesandt. Die Komponenten vom ersten Teil 72 müssen im Autoklaven sterilisierbar sein.

Eine besonders bevorzugte Ausführung des medizintechnischen Messsystems ist, dass das Kamerasystem sternförmig angeordnete oder beabstandete Sensoren aufweist, vor denen jeweils eine Optik angeordnet ist.

Die Kameras können optional mit optischen Hochpassfiltern 104 ausgerüstet sein, sodass nur ein Teil des Lichtspektrums zu den Sensoren gelangt. Dies vereinfacht die Auslegung der Optik.

Anstelle der drei individuellen Kameras kann auch eine gemeinsame Kamera mit entsprechend grossem Bildsensor verwendet werden. Damit werden die drei Bildleiter und Periskope auf den gemeinsamen Bildsensor der einzigen Kamera ausgerichtet. Das Sensorbild enthält in diesem Fall eine Abbildung von allen drei Periskopen. Dies vereinfacht den konstruktiven Aufwand des Kamerasystems.

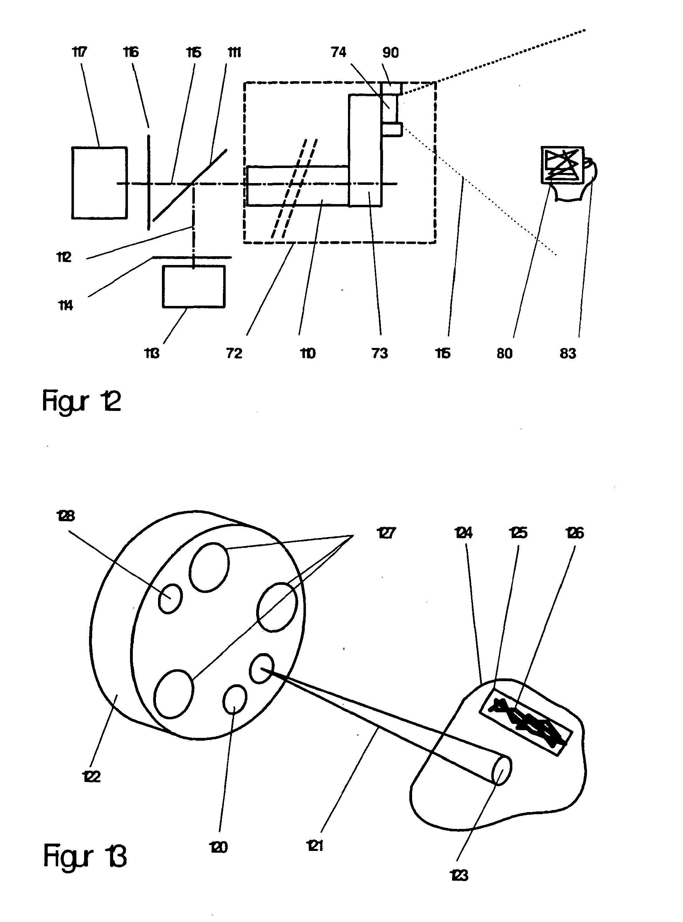

Optional kann, wie in der Figur 12 schematisch und beispielhaft dargestellt, eine Videokamera in die Vorrichtung integriert werden, damit zusätzlich das Messgebiet 115 über eine Periskopoptik 74 eingesehen werden kann. Vorzugsweise wird eine Farb-Videokamera verwendet. In einer möglichen Ausführung der Vorrichtung wird am Ausgang des Bildleiters 110 ausserhalb des sterilen Teils 72 ein Strahlteiler 111 eingefügt. Damit wird ein Teil 112 des Lichts zur Videokamera 113 ausgelenkt. Der optionale Tiefpassfilter 114 schützt den Farbsensor vor infrarotem Licht. Der andere Lichtteil 115 wird via dem optionalen Farbfilter 116 zum Kamerasensor 117 durchgelassen.

Eine andere Variante ist die Verwendung von mindestens zwei Videokameras, was eine Stereobetrachtung des Operationsfeldes ermöglicht.

Eine weitere mögliche Integration von Videokameras ist, dass dafür ein eigenes Periskop verwendet wird. Damit entfällt der Strahlteiler nach dem Bildleiter.

Eine besonders bevorzugte Ausführung des medizintechnischen Messsystems ist, dass es mindestens eine mit dem Kamerasystem starr verbundene Videokamera aufweist.

Wie in der Figur 13 schematisch dargestellt kann ein weiterer Sensor 120 mit eigener Optik und mit optionaler geeigneter Beleuchtung 121 in das Messsystem 122 integriert sein. Die Aufgabe dieses Sensors ist die Messung des Energieflusses wie beispielsweise von sichtbarem Licht oder Wärme von einem bestimmten Gebiet 123 des Objekts. Die Messungen werden in örtlichen Bezug zu der auf dem Objekt 124 angebrachten Struktur 125 und Muster 126 mittels dem Kamerasystem 127 mit optionaler Beleuchtung 128 gebracht.

Diese Messungen mit dem optionalen Sensor sind bevorzugt für berührungslose Diagnostikzwecke geeignet. Die optionale Beleuchtung 121 kann kontinuierlich oder gepulst sein. Das emittierte Spektrum kann im ultravioletten, im sichtbaren, im nahen, mittleren, fernen infraroten und/ oder im Terahertzbereich liegen.

Eine besonders bevorzugte Ausführung des medizintechnischen Messsystems ist, dass es eine mit dem Kamerasystem starr verbundene berührungslose Strahlungsmesseinrichtung für den Bereich von ultraviolettem, sichtbarem, nahem, mittlerem oder fernem infraroten und/oder Terahertz -Spektrumsbereich aufweist.

Das vom Sensor 120 gemessene Spektrum liegt im ultravioletten, im sichtbaren, im nahen, mittleren und/oder fernen infraroten Bereich.

Ein weiterer bevorzugter vom Sensor untersuchter Spektrumsbereich ist derjenige der Terahertzregion.

Die vor dem Sensor angebrachte Optik und der Sensor müssen für das untersuchte Spektrum geeignet sein.

Die vom Sensor empfangene Energie kann vom Objekt selber stammen. In einer bevorzugten Anwendung kann das Objekt dafür mit geeigneten Mitteln für die angewandte Diagnose entsprechend präpariert sein. Beispielsweise können mit spezifischen biologischen Markersubstanzen mögliche Tumorgebiete angereichert werden, die unter entsprechender Beleuchtung vom Sensor 120 erkannt werden. In einer weiteren bevorzugten Anwendung wird das zu untersuchende Gebiet, wie beispielsweise ein Tumorgebiet, mit geeigneten Mitteln präpariert und mittels eingestrahlten elektromagnetischen Wellen lokal erwärmt. Das erwärmte Gebiet wird vom Sensor 120 gemessen und vom Messsystem 122 in Bezug zu den vorhandenen Mustern 126 gesetzt. Es kann auch die zurückgestreute Energie vom von der Strahlungsquelle beleuchteten Objekt sein.

Eine bevorzugte Ausführung des Sensors misst die laterale Ausdehnung des untersuchten Objektoberflächengebietes.

Eine weitere bevorzugte Ausführung misst das zeitliche Verhalten des vom Sensor 120 gemessenen Signals.

Eine weitere bevorzugte Ausführung misst das zeitliche Verhalten des vom Sensor gemessenen Signals bezüglich der eingestrahlten Beleuchtung 121.

Eine weitere bevorzugte Version ist eine Kombination der lateralen und der zeitlichen Messung der von der Objektoberfläche abgestrahlten Energie.

Eine besonders bevorzugte Ausführung des medizintechnischen Messsystems ist, dass es eine mit dem Kamerasystem starr verbundene berührungslose Strahlungsmesseinrichtung für den Bereich von ultraviolettem, sichtbarem, nahem, mittlerem oder fernem infraroten und/oder Terahertz -Spektrumsbereich aufweist.

Eine besonders bevorzugte Ausführung des medizintechnischen Messsystems ist, dass die Strahlungsmesseinrichtung mindestens einen zeit-und/oder ortsauflösenden Sensor aufweist.

Eine besonders bevorzugte Ausführung des medizintechnischen Messsystems ist, dass es mindestens eine mit dem Kamerasystem starr verbundene berührungslose Strahlungseinrichtung für den Bereich von ultraviolettem, sichtbarem, nahem, mittlerem oder fernem infraroten und/oder Terahertz -Spektrumsbereich aufweist.

Ein besonders bevorzugtes Verfahren für einen chirurgischen Eingriff an einem Körperteil mit dem medizintechnischen Messsystem ist, dass berührungslose Diagnoseinformationen lateral und/oder zeitlich aufgelöst in Bezug zur räumlichen Topologie des mit Mustern versehenen Objekts gesetzt sind.

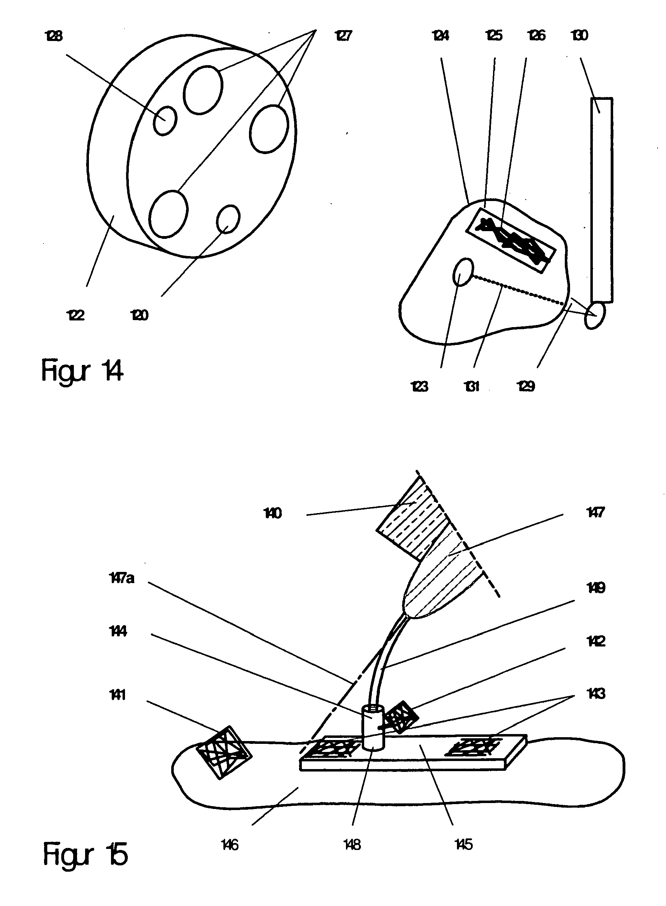

Wie in der Figur 14 schematisch dargestellt wird die Energie 129 in einer weiteren bevorzugten Version der Ausführung hinter dem Objekt 124 mit dem frei beweglichen, händisch geführten Emitter 130 abgestrahlt. Der im Kamerasystem integrierte Sensor 120 misst die vom Gebiet 123 abgestrahlte Energie, die von der eingestrahlten Energie 129 herrührt und entlang der Trajektorie 131 im Objektvolumen modifiziert wurde.

Alternativ emittiert eine im Kamerasystem integrierte Quelle Energie in Richtung des Objekts. Die Intensität des Strahls kann lateral und zeitlich moduliert sein. Ein hinter dem Objekt positionierter Sensor empfängt die durch das Objekt modifizierte Strahlung.

In einer bevorzugten Ausführung wird die gesamte oder ein Teil der Information vom Messsystem und dem optionalen Sensor auf einem Monitor mit Bedienelementen überlagert dargestellt.

Eine bevorzugte Ausführung ist eine Kombination eines Messsystems mit Emitter und Sensor für berührungslose Diagnostikzwecke, die mit medizinischen Behandlungsinstrumenten für Lithotripsie oder RF-Ablation beispielsweise in Weichteilen mechanisch starr verbunden sind und aufgebrachten Mustern auf der Objektoberfläche.

Eine weitere bevorzugte Ausführung ist eine Kombination eines Messsystems mit integriertem RFID Scanner. Damit kann mit auf Instrumenten, Werkzeugen oder Implantaten angebrachten RFID-Transpondern kommuniziert werden.

Eine besonders bevorzugte Ausführung des medizintechnischen Messsystems ist, dass es einen mit dem Kamerasystem starr verbunden RFID Scanner aufweist.

Eine weitere bevorzugte Ausführung eines mobilen Messsystems ist, dass keine Verbindungskabel für Kommunikation und Energie verwendet werden. Alle relevanten Daten für die integrierte Navigation werden vorgängig über abnehmbare Kabel, drahtlos und/oder mit Speicherkarten wie beispielsweise USB Sticks auf das Kamerasystem geladen oder die Messdaten und/oder Videosequenzen dem Host-Rechner übermittelt.

Eine weitere bevorzugte Ausführung eines mobilen Messsystems ohne Kabel für Kommunikation und Energie ist, dass die Vorrichtungen für die Aufhellbeleuchtung nicht aktiviert oder nicht eingebaut ist. Damit wird die lokale Energieversorgung zugunsten einer längeren Betriebsdauer des Kamerasystems entlastet. Eine mögliche Folge davon ist, dass die Belichtungszeit der Kameras länger ist. Damit besteht die Möglichkeit, dass Relativbewegungen zwischen dem Messsystem und dem Objekt unscharfe Bilder zur Folge haben. Unschärfen aufgrund von Kamerasystembewegungen während der Belichtungszeit können mit der Messung und Berücksichtigung von linearen Beschleunigungen und von Winkelbeschleunigungen des Messsystems beispielsweise mit MEMS (MEMS = Micro Electro-Mechanical Systems) kompensiert werden.

Eine besonders bevorzugte Ausführung des medizintechnischen Messsystems ist, dass mit dem Kamerasystem starr verbunden a) eine Energiequelle, b) ein Datenspeicher, c) ein Prozessor, d) eine Anzeige und Bedienelemente sowie e) eine drahtlose Datenübertragungseinrichtung als kompakte Einheit angeordnet sind.

Im folgenden wird in der Figur 15 eine Vorrichtung schematisch vorgestellt, bei der zusätzlich die Lage und Orientierung der individuellen Bohrhülsen bezüglich Referenzmustern vom Kamerasystem erfasst werden.

Die Vorrichtung basiert auf einem optischen Kamerasystem 140 und Mustern 141, 142, 143 anstelle der Lokatoren. Dabei ist das Kamerasystem 140 fähig, die Position und Orientierung der Muster zu messen. An jede der Hülsen 144, die am Implantat 145 befestigt ist, ist eine Struktur mit einem Muster 142 starr angeordnet. Optional kann das Muster in die Hülsenoberfläche integriert sein.

Das Implantat 145 selber kann ebenfalls solche Muster 143 aufweisen, entweder in die Oberfläche integriert oder in Form einer abnehmbaren Struktur.

An der Knochenoberfläche 146 sind Muster 141 temporär angebracht. Damit werden die Orientierungen und Positionen der Hülsen, des Implantats und des Knochenteils vom mobilen Messsystem 140, das am Bohrgerät 147 mit der Werkzeugachse 147a fixiert ist, permanent gemessen. Mit dieser Vorrichtung ist der Chirurg in der Lage, die Bohrlöcher 148 im Knochen auch mit biegsamem Bohrer 149 exakt zu machen.

Ein offenbartes Verfahren für einen chirurgischen Eingriff an einem Körperteil mit dem medizintechnischen Messsystem ist, dass ein Implantat und/oder eine Prothese, optional ein oder mehrere angeordnete Bohrhülsen und der zu behandelnde Knochen oder Knochenteile mit geometrischen Mustern ausgestattet sind und dass die räumliche Lage und Orientierung eines Bohrloches bezüglich dem Knochen vom Messsystem gemessen werden.

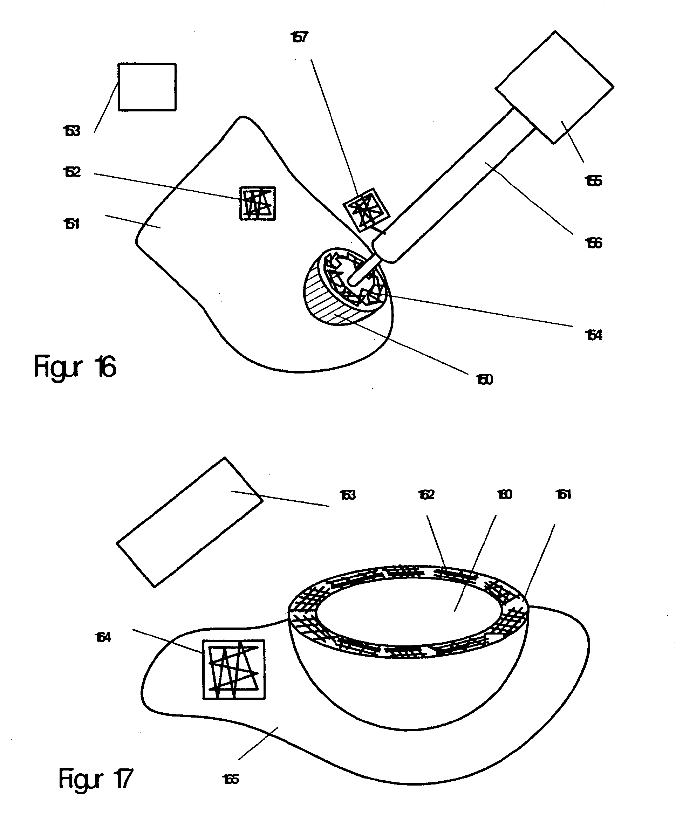

Beim Implantieren von Gelenkpfannen - Prothesen muss streng darauf geachtet werden, dass die Prothese optimal im Beckenknochenmaterial gesetzt wird. In der Figur 16 wird schematisch dargestellt wie die Lage und Orientierung eines dafür eingesetzten Fräskopfs 150 relativ zum im Beckenknochen (Pelvis) 151 verankerten, registrierten Muster 152 mit einem mobilen Messsystem 153 gemessen wird. Die Geometrie des Fräskopfs 150 und das Muster 154 sind, definiert beispielsweise gemäss Herstellung, zueinander bekannt. Der Fräskopf 150 mit dem Muster 154 ist in den Schaft 156 der Bohrmaschine 155 eingespannt. Das Messsystem 153 misst simultan die beiden Marker 152 und 154. Damit sind jederzeit die Lage und Orientierung des Fräskopfs 150 relativ zum Muster 152 auf dem Pelvis bekannt. Diese Messung kann beispielsweise nach jedem Fräsarbeitsschritt (mit wenig Materialaushub) durchgeführt werden, bis die Ausfräsung optimal im Knochenmaterial liegt.

In einer bevorzugten Variante besitzt der Fräskopf 150 kein Muster. Dafür wird das Muster 157, das beispielsweise auf dem Schaft 156 der Bohrmaschine 155 fix montiert ist, simultan mit dem registrierten Muster 152 auf dem Pelvis 151 vom Messsystem 153 gemessen. Die Lage des Fräskopfs 150 wird dabei vorgängig bezüglich dem Muster 157 kalibriert.

Im folgenden wird eine Vorrichtung und Methode vorgestellt, bei der die Lage und die Orientierung beispielsweise von implantierten Gelenkpfannen-Prothesen 160 ermittelt wird. Wie in der Figur 17 schematisch dargestellt, ist der Pfannenrand 161 mit einem geeigneten Muster 162 versehen, das vom vorzugsweise mobilen Messsystem 163 erkannt und analysiert wird. Ein weiteres Muster 164 als Referenz ist beispielsweise auf dem Beckenknochen (Pelvis 165) angebracht. Damit ist die Position und Orientierung der Gelenkpfannen-Prothese bezüglich zum Referenzmuster auf dem Pelvis messbar. Weitere Musterteile 162 beinhalten eine Identifikation und/oder Informationen zur Geometrie der Prothese wie Durchmesser und Zentrum der Kugelschale.

Optional kann mittels dem strukturieren Licht die Oberflächentopologie der Prothese bezüglich dem Referenzmuster gemessen werden.

Ein offenbartes Verfahren für einen chirurgischen Eingriff an einem Körperteil mit dem medizintechnischen Messsystem ist, dass die flächenhaften Muster direkt an geeigneter Stelle auf ein Implantat für die Bestimmung der räumlichen Lage und Orientierung und/oder für die Identifizierung und/oder geometrische Charakterisierung angebracht sind.

Die in der Patentschrift US6424856B1 beschriebene Methode, bei der CAS für Weichteile angewendet werden kann, wird mit den im folgenden beschriebenen Massnahmen im Hinblick auf die praktische Realisierung wesentlich verbessert.

Die Massnahmen sind erstens eine ausreichende Überdeckung der Weichteiloberfläche mit einem geeigneten feinen Muster mit ausreichender Redundanz auf flexibler Folie und zweitens wird das mobile Kamerasystem relativ nahe an das Weichteil positioniert.

In einer bevorzugten Anwendung sind die Messungen in bestimmten Arbeitsschritten in Echtzeit verfügbar. Damit wird das Messsystem für Navigationsaufgaben eingesetzt.

In einer weiteren bevorzugten Anwendung misst in bestimmten Arbeitsschritten das Messsystem sequenziell die Topologie der Oberfläche.

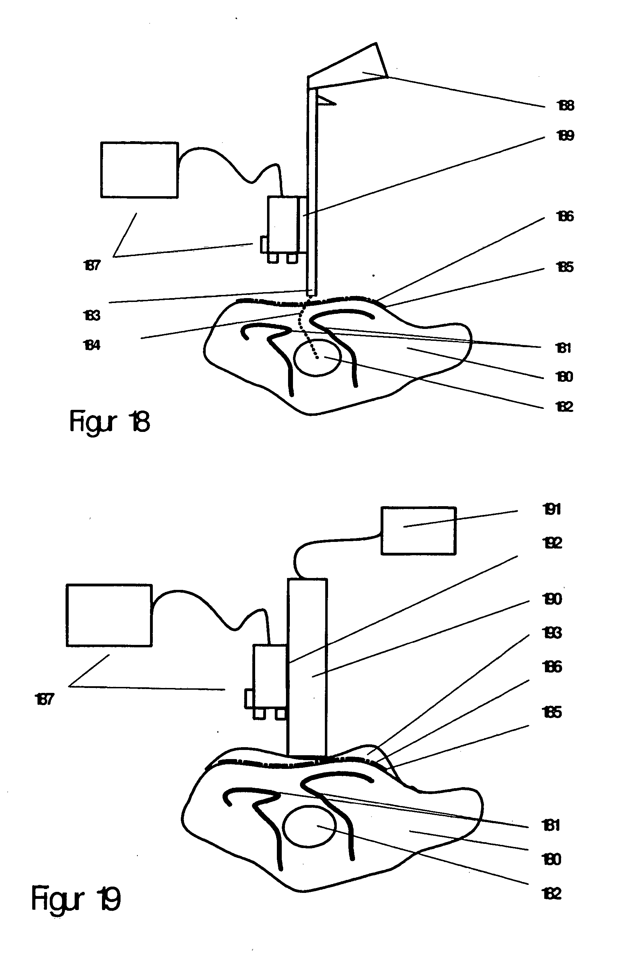

Eine Vorrichtung und eine Methode für beispielsweise eine Tumorentfernung aus der freigelegten Leber ist schematisch in Figur 18 dargestellt. Figur 18 stellt ein Schnittbild durch die Leber 180 schematisch dar. Dabei sind die Gefässe 181 und der Tumor 182 sichtbar.

Die Topologie der Leber wird präoperativ beispielsweise mittels Röntgentechnik und gegebenenfalls Kontrastmittel und/oder Ultraschallmessungen und/oder MRI ermittelt. Der Chirurg will die Instrumentenspitze 183 so durch das Lebergewebe entlang dem optimalen Pfad 184 zum Tumor 182 führen, dass dabei beispielsweise keine wichtigen Organgefässe 181 verletzt werden. Beim heutigen Stand der Technik benutzt der Chirurg während dem Eingriff an der freigelegten Leber Ultraschalltechnik, die oft nicht ergonomisch eingesetzt werden kann, um die Instrumentenspitze zu führen.

Bei der vorgestellten Vorrichtung werden auf die Oberfläche der freigelegten Leber an einigen Stellen beispielsweise dehnbare Folien 185 mit aufgebrachten kontrastreichen geometrischen Mustern 186 angebracht.

Damit können mit dem Kamerasystem Messungen der Oberflächentopologie von mit flexiblen Mustern versehenen deformierbaren, flexiblen Oberflächen durchgeführt werden: an der CARS 2008 in Barcelona, aber auch in der Patenschrift US6424856B1 von Brainlab werden eine Methoden vorgestellt, bei der die dynamischen Volumeneigenschaften auf Grund von äusseren Krafteinwirkungen auf beispielsweise ein Organs mit Tumor modelliert werden können. Dabei werden die gemessenen, geometrischen Veränderungen der flexiblen Oberfläche des Organs auf Grund von punktuellen Krafteinwirkungen (beispielsweise verursacht durch das oben beschriebene Instrument) und bekannte Materialeigenschaften des Organs verwendet.

Die Muster oder Teile davon werden vom mobilen, optischen Kamerasystem 187 gemessen. Damit sind die Lage und die Orientierung des Kamerasystems bezüglich den Mustern bekannt. Werden das Kamerasystem 187 und das chirurgische Instrument 188 starr mittels dem Adapter 189 miteinander verbunden, sind Lage und Orientierung des Instruments bezüglich dem Muster 186 ebenfalls bekannt.

Nach Durchführung der Registrierung, wie weiter unten dargestellt, sind die Lagen und Orientierungen der Muster bezüglich der Lebertopologie ausreichend bekannt. Somit kann der Chirurg das Instrument zu Beginn gezielt in die Leber einfahren und entlang dem optimalen Pfad 184 an den Blutgefässen und anderen wichtigen Gewebeteilen vorbei zur gewünschten Stelle des Tumors führen. Anschliessend findet die eigentliche chirurgische Behandlung beispielsweise mit RF-Ablation des Tumors statt. Nach dem chirurgischen Eingriff werden die Folien mit Muster wieder entfernt. Alternativ verwendete bioresorbierbare Folien müssen nicht entfernt werden.

In Figur 19 wird beispielhaft dargestellt, wie das auf der Leberoberfläche angebrachte Muster bezüglich der Leber registriert wird. Das mobile Kamerasystem 187 wird mit dem Messkopf 190 des Ultraschallgeräts 191 starr mittels einer geeigneten Vorrichtung 192 verbunden. Die Messdaten sowohl vom Ultraschallgerät als auch vom optischen Kamerasystem werden erfasst und mittels geeigneter Software aufeinander abgestimmt. Somit ist die Lage der Muster bezüglich der Topologie des Lebervolumens bekannt.

Diese Methode wird beispielsweise dadurch ergänzt, dass die Folie 185 mit den Mustern zusätzliche definierte Gebiete enthält, die ein kontrastreiches Bild bei Ultraschallmessungen, gegebenenfalls unter Einsatz eines Koppelmediums 193, ergeben.

Eine weitere Methode für die Registrierung der Muster bezüglich der Lebertopologie sieht vor, dass die Folien mit den kontrastreichen Mustern auch röntgen-opake Gebiete enthalten. Damit werden mittels Röntgentechnik in der Leber die röntge-opaken Gebiete und damit die kontrastreichen Muster auf der Folie bezüglich dem Volumen der Leber registriert.

Chirurgische, navigierte Eingriffe mit oben beschriebener Technologie eignen sich auch für nichtstarre Objekte wie Organe, Muskeln und/oder Bänder. Mit einer weiteren bevorzugten Anwendung des beschriebenen Messsystems werden Messung mit strukturiertem Licht für die Ermittlung individueller Oberflächen-Topologien mehrerer Objekte, die optional mit starren und/oder flexiblen Mustern ausgestattet sind, im Operationsgebiet durchgeführt.

Beispielsweise ist diese bevorzugte Anwendung die kontrollierte, optimale Verschiebung von Körperteilen zueinander im Gebiet der maxillofazialen Chirurgie.

Ein besonders bevorzugtes Verfahren für einen chirurgischen Eingriff an einem Körperteil mit dem medizintechnischen Messsystem ist, dass a) mittels eines bildgebenden Verfahrens wie MRI, Ultraschall- oder Röntgentechnik das Volumen des Körperteils dreidimensional erfasst wird, b) die Lage von Körperteilen, wie Blutgefässen, Organen, Drüsen und Tumoren dreidimensional beschrieben wird, c.) die Arbeitsschritte des chirurgischen Eingriffs definiert werden, d.) beim chirurgischen Eingriff das Körperteil in einem ersten Arbeitsschritt freigelegt wird, e.) starre Strukturen oder flexible Folien mit geometrischen Mustern erkennbar für das optische Kamerasystem und sekundäre Muster, deren Lage bezüglich den geometrischen bekannt und erkennbar für Ultraschallmessungen, CT und/oder C-Bogen sind, auf der Oberfläche des freigelegten Körperteils angebracht werden, f.) das geometrische Muster mittels Messung mit Ultraschall, CT und/oder C-Bogen bezüglich dem sekundären Muster auf der Oberfläche des Körperteils und/oder dem Volumen des Körperteils unter Verwendung des vorgängig erstellten Models registriert wird, g.) Bestimmung der optimale Pfad eines chirurgischen Instruments aufgrund der erfassten Volumentopologie bestimmt wird und h.) ein mit Mustern ausgestattetes chirurgisches Instrument vom Messsystem geführt bezüglich den geometrischen Mustern auf der Folie an anderen Körperteilen, wie Arterien und Venen vorbei auf optimalem Pfad zur zu behandelnden Stelle vorgeschoben und eingesetzt wird.

Ein besonders bevorzugtes Verfahren für einen chirurgischen Eingriff an einem Körperteil mit dem medizintechnischen Messsystem ist, dass das chirurgische Instrument für die Hochfrequenzablation (RF-Ablation) verwendet wird.

Ein besonders bevorzugtes Verfahren für einen chirurgischen Eingriff an einem Körperteil mit dem medizintechnischen Messsystem ist, dass der Eingriff an einer Leber durchgeführt wird.

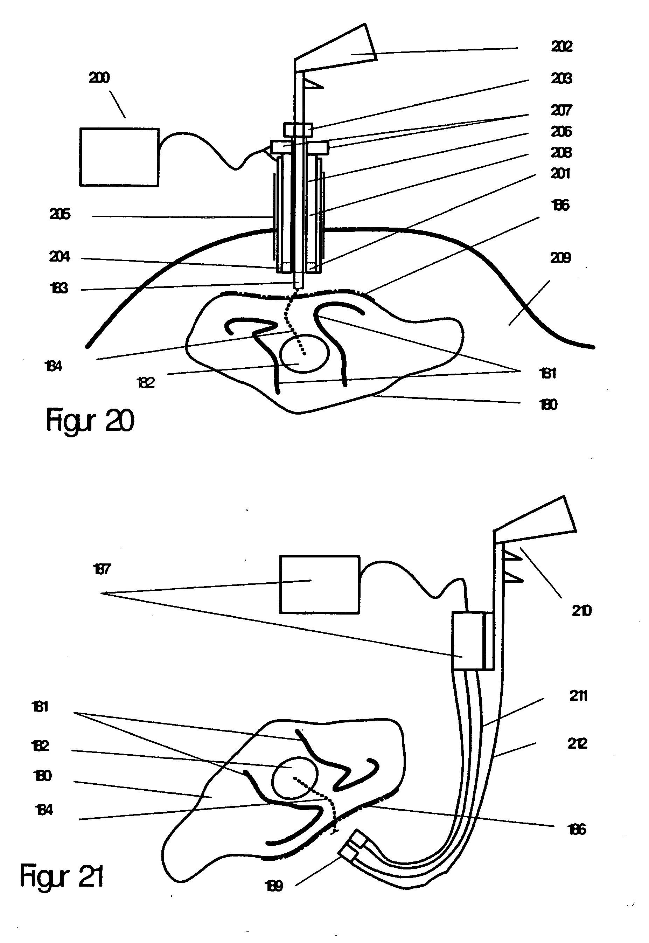

In der Figur 20 wird die Vorrichtung beispielhaft für die laparoskopische Chirurgie gezeigt. Auch in diesem Fall soll beispielsweise ein Tumor aus der Leber entfernt werden.

Das Kamerasystem 200 hat mindestens zwei (vorteilhaft drei) endoskopartige Optiken 201, die um das Instrument 202 beispielsweise kreisförmig starr angeordnet sind. Die Sensoren 207 sind über die Bildleiter 208 mit den Optiken 201 verbunden. In einer weitem bevorzugten Ausführung der Vorrichtung sind die Optiken, Sensoren und ein Teil der Elektronik vorne am Endoskop angebracht.

Eine besonders bevorzugte Ausführung des medizintechnischen Messsystems ist, dass das Kamerasystem mindestens zwei mit Flächensensoren ausgestattete Endoskope aufweist, deren Optiken starr miteinander verbunden sind.

Das Instrument kann seine Lage und Orientierung bezüglich dem Kamerasystem verändern. Die Lage und eventuell die Orientierung des Instruments im Führungsrohr 206 bezüglich den Optiken wird beispielsweise mit einem Positionssensor 203 erfasst.

Mittels der Beleuchtungsvorrichtung 204 wird für eine ausreichende Ausleuchtung in der Bauchhöhle 209 gesorgt. Das äussere Rohr 205 dient als Abdichtung.

Damit ergibt sich ein ausreichend schlankes Gebilde, das durch das äussere Rohr 205 in die Bauchhöhle 209 vorgeschoben wird.

Auch in diesem Fall werden beispielsweise Folien mit Muster 186 an die Leberoberfläche 180 befestigt. Die Registrierung erfolgt beispielsweise mittels Röntgentechnik und röntgen-opaken Gebieten auf der Folie.

Somit kann der Chirurg das Instrument zu Beginn gezielt in die Leber einfahren und dann entlang dem optimalen Pfad 184 an den Blutgefässen und anderen wichtigen Gewebeteilen vorbei zur gewünschten Stelle des Tumors führen. Anschliessend findet die eigentliche chirurgische Behandlung des Tumors statt. Nach dem chirurgischen Eingriff werden die Folien mit Muster wieder entfernt. Alternativ verbleiben bioresorbierbare Folien ganz oder teilweise im Körper und lösen sich im Laufe der Zeit ohne Nachteil für den Patienten auf.

Chirurgische Eingriffe mit flexiblen Endoskopen / Laparoskopen eignen sich genau so gut für die vorgängig beschriebene Organbehandlung mit Navigation. Dabei führt der Operateur das flexible Rohr derart in den Patienten ein, dass er vorzugsweise ein Organ auch von einer nicht direkt zugänglichen Seite behandeln kann. Er bringt an der gewünschten Stelle die Oberflächenmarkierungen an dem Organ an, dann ermittelt er mit einem bildgebenden System wie Röntgen, US oder MRI die Volumendaten des Organs gemeinsam mit den Markierungen, dann führt er das Arbeitsinstrument durch das Organ an die gewünschte Stelle. Lage- und Formveränderungen des Organs sind über die angebrachten Markierungen jederzeit erfassbar und für die exakte Navigation vorhanden.

Eine weitere bevorzugte Vorrichtung mit endoskopartigen Kamerasystemen 187, 189, 211 mit mindestens zwei Kameras ist beispielhaft in der Figur 21 schematisch dargestellt. Dabei wird der vordere Teil des endoskopartigen Kamerasystems 189 mit geeigneten Techniken 212 um das zu behandelnde Organ 180 kontrolliert mittels dem Steuerteil 210 herumgeführt, so dass das Kamerasystem die Seitenwand oder die Hinterseite des Organs einsehen kann. Geeignete Muster 186 sind auf der Organoberseite an geeigneter Stelle auf dem Organ angebracht. Eine Registrierung wird beispielsweise mittels Röntgentechnik oder mittels Ultraschall durchgeführt. Damit kann die mit den Mustern versehene Oberfläche in Relation zum Organinneren mit beispielsweise einem Tumor 182 und/oder Blutgefässen 181 dreidimensional registriert werden. Diese Vorrichtung gestattet räumliches Navigieren hinter Organen oder anderen nicht direkt zugänglichen Körperstellen von mit geeigneten Markern ausgestatteten chirurgischen Instrumenten.

Eine besonders bevorzugte Verwendung des medizintechnischen Messsystems für einen chirurgischen Eingriff an einem Körperteil ist für eine Laparoskopie.

Es zeigt
- Figur 1: schematisch eine Anordnung von einem Bohrprozess mittels einer Führungshülse,
- Figur 2a: schematisch eine Struktur mit einfachem dreidimensionalem Muster ohne optionale Schutzschicht,
- Figur 2b: schematisch eine Struktur mit einfachem planarem Muster ohne optionale Schutzschicht,
- Figur 2c: schematisch eine Struktur mit einfachem, dreidimensionalem Muster mit transparenter Schutzschicht,
- Figur 2d: schematisch eine Struktur mit dreidimensionaler Struktur in Form einer stumpfen Pyramide,
- Figur 3: schematisch eine Struktur mit optisch kontrastreichen geometrischen Mustern auf sehr flexiblen, dehnbaren Streifen auf deformierbarem Objekt,
- Figur 4: schematisch eine andere Struktur mit optisch kontrastreichen, geometrischen Mustern auf sehr flexiblen, dehnbaren Folien auf deformierbarem Objekt,
- Figur 5: schematisch die wesentlichen Komponenten einer aktiven Struktur,
- Figur 6: schematisch eine konfigurierbare aktive Struktur mit Messsystem,
- Figur 7: schematisch ein Messsystem mit mehreren Kameras und mit integrierter Lichtquelle für die Projektion von strukturiertem Lichtmuster auf mit Strukturen ausgerüstete Objekte,
- Figur 8: schematisch eine Anordnung eines optischen Messsystem mit drei Kameras ausgerüstet mit Periskopen,
- Figur 9: schematisch Details einer Kamera mit Periskop,
- Figur 10: schematisch eine Anordnung eines optischen Messsystem mit sterilisierbarer Optik, Sensoren und Elektronikteilen,
- Figur 11: schematisch Details einer Kamera mit sterilisierbarer Optik, Sensoren und Elektronikteilen,
- Figur 12: schematisch eine Anordnung eines Kamerasystems mit integrierter Videokamera,
- Figur 13: schematisch eine Anordnung eines Kamerasystems mit integriertem Emitter und Sensor für berührungslose Diagnostik des Objekts,
- Figur 14: schematisch eine Anordnung eines Kamerasystems mit Emitter hinter dem Körper und im Kamerasystem integriertem Sensor für Diagnostik,
- Figur 15: schematisch ein mobiles, an einem Bohrgerät angebrachtes Kamerasystem, welches die relativen Orientierungen und Positionen von mit Mustern ausgerüsteten Hülsen, Plattenimplantaten und Knochen misst,
- Figur 16: schematisch ein Fräskopf mit Muster, dessen Lage und Position relativ zum Referenzmuster auf dem Pelvis vom Messsystem gemessen werden,
- Figur 17: schematisch eine implantierte Gelenkpfanne mit Muster, dessen Lage und Position relativ zum Referenzmuster auf dem Pelvis vom Messsystem gemessen werden,
- Figur 18: schematisch eine Vorrichtung und eine Methode für eine Tumorentfernung beispielsweise aus der freigelegten Leber,
- Figur 19: schematisch die Registrierung des auf der Leberoberfläche angebrachten Musters bezüglich der Leber mittels Ultraschalltechnik,
- Figur 20: schematisch eine Vorrichtung für die laparoskopische Chirurgie,
- Figur 21: schematisch eine Vorrichtung für die laparoskopische Chirurgie mit flexiblen Endoskopen.

## Patentansprüche

1. Mobiles, medizintechnisches Messsystem (60) zum Vermessen einer Oberfläche eines verformbaren Körperteils (67) mit einer Kombination aus einem optischen Kamerasystem (63) und einer Lichtquelle (64) für die Projektion von Licht (66) auf die Oberfläche des Körperteils (67), wobei das Kamerasystem (63) mindestens zwei flächenhafte, bildgebende Sensoren aufweist und geeignet ist, das von der Oberfläche des verformbaren Körperteils (67) gestreute Licht (69) zu messen, wobei an der Oberfläche des verformbaren Körperteils eine flexible Struktur mit einem flächenhaften, optisch kontrastreichen, geometrischen und redundanten Muster (21, 22, 23) angebracht ist, ***dadurch gekennzeichnet, dass*** die flexible Struktur eine auf die Oberfläche des verformbaren Körperteils aufbringbare dehnbare Folie ist.

2. Medizintechnisches Messsystem nach Anspruch 1, ***dadurch gekennzeichnet, dass*** für eine Projektion von strukturiertem Licht auf die Oberfläche (69) Laserlicht auf diffraktive optische Elemente (DOE) projiziert wird.

3. Medizintechnisches Messsystem nach einem der vorhergehenden Ansprüche, ***dadurch gekennzeichnet, dass*** es eine Kombination aus berührungsloser Strahlungseinrichtung und berührungsloser Strahlungsmesseinrichtung für den Bereich von ultraviolettem, sichtbarem, nahem, mittlerem oder fernem infraroten und/oder Terahertz-Spektrumsbereich aufweist.

4. Medizintechnisches Messsystem nach einem der vorhergehenden Ansprüche, ***dadurch gekennzeichnet, dass*** es mindestens eine mit dem Kamerasystem (63) starr verbundene Videokamera aufweist.

5. Medizintechnisches Messsystem nach einem der vorhergehenden Ansprüche, ***dadurch gekennzeichnet, dass*** das Kamerasystem (63) mindestens zwei mit Flächensensoren ausgestattete Endoskope aufweist, deren Optiken starr miteinander verbunden sind.

6. Medizintechnisches Messsystem nach einem der vorhergehenden Ansprüche, ***dadurch gekennzeichnet, dass*** mit dem Kamerasystem (63) starr verbunden a) eine Energiequelle, b) ein Datenspeicher, c) ein Prozessor, d) eine Anzeige und Bedienelemente sowie e) eine drahtlose Datenübertragungseinrichtung als kompakte Einheit angeordnet sind.

7. Medizintechnisches Messsystem nach einem der vorhergehenden Ansprüche ***dadurch gekennzeichnet, dass*** die Struktur eine Kombination mit starren und flexiblen Gebieten aufweist.

8. Medizintechnisches Messsystem nach einem der vorhergehenden Ansprüche, ***dadurch gekennzeichnet, dass*** das mobile Kamerasystem (63) mit einem medizinischen Instrument oder Werkzeug verbunden ist.

9. Medizintechnisches Messsystem nach einem der vorhergehenden Ansprüche, ***dadurch gekennzeichnet, dass*** die Struktur geometrische und sekundäre, von nichtoptischen bildgebenden Systemen wie einem Ultraschallgerät, MRI und/oder Röntgensystem erkennbare Muster aufweist.

10. Verfahren zum Betreiben eines medizintechnischen Messsystems (60) nach einem der vorhergehenden Ansprüche zum Vermessen der Oberfläche (69) und der Oberflächenformänderungen eines verformbaren Körperteils (67), **dadurch gekennzeichnet, dass** die Messungen von auf die Oberfläche (69) des verformbaren Körperteils (67) projiziertem strukturiertem Licht, auf das Muster (21, 22,23) auf der flexiblen Struktur (20, 61), die an der Oberfläche des verformbaren Körperteils (67) angebracht ist, räumlich und zeitlich bezogen werden.

11. Verfahren nach Anspruch 10, ***dadurch gekennzeichnet, dass*** die mittels strukturierten Lichts vermessene Körperoberfläche bezüglich den mit anderen bildgebenden Verfahren wie MRI, Ultraschall- oder Röntgentechnik erfassten Körperdaten registriert wird.

12. Verfahren nach einem der vorhergehenden Verfahrensansprüche, ***dadurch gekennzeichnet, dass*** berührungslose Diagnoseinformationen lateral und/oder zeitlich aufgelöst in Bezug zur räumlichen Topologie des mit Mustern versehenen Objekts gesetzt werden.

## Claims

1. A mobile medical-technical measuring system (60) for measuring a surface of a deformable body part (67) with a combination of an optical camera system (63) and a light source (64) for the projection of light (66) onto the surface of the body part (67), the camera system (63) having at least two laminar imaging sensors and being suitable for measuring the light (69) scattered by the surface of the deformable body part (67), a flexible structure with a laminar, optically high-contrast, geometric and redundant pattern (21, 22, 23) being attached to the surface of the deformable body part, ***characterised in that*** the flexible structure is an expandable film that can be attached to the surface of the deformable body part.

2. The medical-technical measuring system according to Claim 1, ***characterised in that,*** for a projection of structured light onto the surface (69), laser light is projected onto diffractive optical elements (DOEs).

3. The medical-technical measuring system according to any one of the preceding claims, ***characterised in that*** it has a combination of contactless radiation arrangement and contactless radiation measuring arrangement for the ultraviolet, visible, near, mid or far infrared and/or terahertz region of the spectrum.

4. The medical-technical measuring system according to any one of the preceding claims, ***characterised in that*** it has a video camera connected rigidly to the camera system (63).

5. The medical-technical measuring system according to any one of the preceding claims, ***characterised in that*** the camera system (63) has at least two endoscopes equipped with surface sensors, the lenses of said endoscopes being rigidly interconnected.

6. The medical-technical measuring system according to any one of the preceding claims, ***characterised in that*** a) a power source, b) a data memory, c) a processor, d) a display and operating elements, and e) a wireless data transmission arrangement are rigidly connected to the camera system (63) and are arranged as a compact unit.

7. The medical-technical measuring system according to any one of the preceding claims, ***characterised in that*** the structure has a combination with rigid and flexible areas.

8. The medical-technical measuring system according to any one of the preceding claims, ***characterised in that*** the mobile camera system (63) is connected to a medical instrument or tool.

9. The medical-technical measuring system according to any one of the preceding claims, ***characterised in that*** the structure has geometric and secondary patterns that can be recognised by non-optical imaging systems, such as an ultrasound device, MRI and/or X-ray system.

10. A method for operating a medical-technical measuring system (60) according to any one of the preceding claims for measuring the surface (69) and the changes in surface form of a deformable body part (67), ***characterised in that*** the measurements of structured light projected onto the surface (69) of the deformable body part (67) are put in spatial and temporal relation with the pattern (21, 22, 23) on the flexible structure (20, 61) attached to the surface of the deformable body part (67).

11. The method according to Claim 10, ***characterised in that*** the body surface measured by means of structured light is recorded with respect to the body data ascertained using other imaging methods, such as MRI, ultrasound technology or X-ray technology.

12. The method according to any one of the preceding method claims, ***characterised in that*** laterally and/or time resolved contactless diagnosis information is put in relation with the spatial topology of the object provided with patterns.

## Revendications

1. Système de mesure mobile de technique médicale (60) pour la mesure d'une surface d'un élément corporel (67) déformable avec une combinaison formée par un système de caméra optique (63) et une source lumineuse (64) pour la projection de lumière (66) sur la surface de l'élément corporel (67), le système de caméra (63) présentant au moins deux capteurs imageurs surfaciques et convenant pour mesurer la lumière diffusée (69) par la surface de l'élément corporel (67) déformable, moyennant quoi une structure flexible avec un motif (21, 22, 23) surfacique, riche en contrastes du point de vue optique, géométrique et redondant est placée sur la surface de l'élément corporel déformable, ***caractérisé en ce que*** la structure flexible est un film extensible pouvant être appliqué sur la surface de l'élément corporel déformable.

2. Système de mesure de technique médicale selon la revendication 1, ***caractérisé en ce que,*** pour une projection de lumière structurée sur la surface (69), on projette de la lumière laser sur des éléments optiques diffractifs (DOE).

3. Système de mesure de technique médicale selon l'une des revendications précédentes, ***caractérisé en ce qu'il*** présente une combinaison formée par un dispositif d'irradiation sans contact et un dispositif de mesure d'irradiation sans contact pour la plage de la plage spectrale de l'ultraviolet, de l'infrarouge visible, proche, moyen ou lointain et/ou térahertzienne.

4. Système de mesure de technique médicale selon l'une des revendications précédentes, ***caractérisé en ce qu'il*** présente au moins une caméra vidéo reliée de manière rigide au système de caméra (63).

5. Système de mesure de technique médicale selon l'une des revendications précédentes, ***caractérisé en ce que*** le système de caméra (63) présente au moins deux endoscopes munis de capteurs plans, dont les optiques sont reliées de manière rigide entre elles.

6. Système de mesure de technique médicale selon l'une des revendications précédentes, ***caractérisé en ce que,*** sont disposés de manière reliée de façon rigide au système de caméra (63), a) une source d'énergie, b) une mémoire de données, c) un processeur, d) un affichage et des éléments de commande ainsi que e) un dispositif de transmission de données sans fil, en tant qu'une unité compacte.

7. Système de mesure de technique médicale selon l'une des revendications précédentes, ***caractérisé en ce que*** la structure présente une combinaison avec des zones rigides et flexibles.

8. Système de mesure de technique médicale selon l'une des revendications précédentes, ***caractérisé en ce que*** le système de caméra mobile (63) est en liaison avec un instrument ou outil médical.

9. Système de mesure de technique médicale selon l'une des revendications précédentes, ***caractérisé en ce que*** la structure présente des motifs géométriques et secondaires pouvant être reconnus par des systèmes imageurs non optiques tels un appareil à ultrasons, un appareil d'IRM et/ou un système radiologique.

10. Procédé pour exploiter un système de mesure de technique médicale (60) selon l'une des revendications précédentes pour mesurer la surface (69) et les modifications de forme de la surface d'un élément corporel (67) déformable, ***caractérisé en ce que*** les mesures de lumière structurée projetée sur la surface (69) de l'élément corporel (67) déformable sont mises en rapport avec le motif (21, 22, 23) sur la structure flexible (20, 61), laquelle est appliquée sur la surface de l'élément corporel (67) déformable, de manière spatiale et temporelle.

11. Procédé selon la revendication 10, ***caractérisé en ce que*** la surface corporelle mesurée à l'aide de lumière structurée est enregistrée en rapport avec les données sur le corps détectées à l'aide d'autres procédés imageurs tels l'IRM, la technique des ultrasons ou des rayons X.

12. Procédé selon l'une des revendications précédentes concernant le procédé, ***caractérisé en ce que*** des informations de diagnostic sans contact sont mises en rapport avec la topologie spatiale de l'objet muni de motifs d'une manière résolue latéralement et/ou dans le temps.
